# EUROPEAN PATENT APPLICATION

(11) **EP 4 104 856 A1**
(43) Date of publication of application: **21.12.2022**
(21) Application number: 21753897.4
(22) Date of filing: 10.02.2021
(51) Int. Cl.: A61K 39/395, C07K 16/28, A61P 35/00

(54) **USE OF ANTI-PD-1 ANTIBODY IN TREATMENT OF TUMORS**

(30) Priority: 13.02.2020 CN 202010090829
(71) Applicant: Shanghai Junshi Biosciences Co., Ltd., Pilot Free Trade Zone Shanghai 201210 (CN)
(72) Inventor: YAO, Sheng, Shanghai 201210 (CN); FENG, Hui, Shanghai 201210 (CN); WU, Hai, Shanghai 201210 (CN)
(74) Representative: V.O.
(86) International application number: PCT/CN2021/076456
(87) International publication number: WO 2021/160151

(57) **Abstract**

The present invention relates to use of an anti-PD-1 antibody in the treatment of melanoma. The present invention also relates to use of an agent for detecting *BRAF, NRAS,* and *CDK4*/*CCND1* gene mutations in a detection kit for predicting the therapeutic effect of an anti-PD-1 antibody and/or an antigen-binding fragment thereof administered alone to a patient with melanoma.

## Description

### TECHNICAL FIELD

The present invention relates to use of an anti-PD-1 antibody in the treatment of a tumor. In particular, the present invention relates to use of the anti-PD-1 antibody in the treatment of melanoma; and use of the anti-PD-1 antibody in the preparation of a medicament for treating melanoma; and a method for predicting the therapeutic effect of the anti-PD-1 antibody administered alone on melanoma using a biomarker.

### BACKGROUND

Immune escape is one of the characteristics of cancer. Ahmadzadeh, M. et al disclosed in Blood, 114: 1537-44 that tumor-specific T lymphocytes are often present in the tumor microenvironment, draining lymph nodes and peripheral blood, but are generally unable to control tumor progression due to the network of immunosuppressive mechanisms present in the tumor microenvironment. CD8⁺ tumor infiltrating T lymphocytes (TILs) generally express activation-induced inhibitory receptors, including CTLA-4 and PD-1, while tumor cells often express immunosuppressive ligands, including PD-1 ligand 1 (PD-L1, also called B7-H1 or CD274), which inhibits activation and effector functions of T cells. In the inhibitory mechanism, PD-1 and its ligands have become an important pathway for tumor cells to suppress activated T cells in the tumor microenvironment.

Programmed death receptor 1 (PD-1) plays an important role in immune regulation and maintenance of peripheral tolerance. PD-1 is expressed primarily in activated T and B cells and functions to inhibit the activation of lymphocytes, which is a normal peripheral tissue tolerance mechanism of the immune system that prevents over-reactive immunity. However, the activated T cells infiltrated in the tumor microenvironment highly express PD-1 molecules, and inflammatory factors secreted by the activated leukocytes can induce the tumor cells to highly express ligands PD-L1 and PD-L2 of PD-1, resulting in the continuous activation of the PD-1 pathway of the activated T cells in the tumor microenvironment, and the suppression of T cell function to kill tumor cells. Therapeutic anti-PD-1 antibodies can block this pathway, partially restore the function of T cells, and enable the activated T cells to continuously kill tumor cells.

Blocking the PD-1/PD-L1 pathway has proven to be an effective way to induce a durable anti-tumor response in various cancer indications over the last decade. Monoclonal antibodies (mAbs) blocking the PD-1/PD-L1 pathway can enhance activation and effector functions of tumor specific T cells, reduce tumor burden, and improve survival rate. Between 2014 and 2017, the FDA has approved 2 anti-PD-1 monoclonal antibodies (nivolumab and pembrolizumab) and 3 anti-PD-L1 monoclonal antibodies (atezolizumab, avelumab and durvalumab) for treating human tumors. Melanoma was the first indication for which nivolumab and pembrolizumab were approved in 2014.

Melanoma has long been recognized as a more immunogenic cancer species because of the frequent observation of lymphocyte infiltration into the tumor and clinical response to high-dose IL-2 immunotherapy. Mechanistically, chronic ultraviolet radiation exposure associated with induction of DNA damage is a leading cause of melanoma in western populations. Chronic sun-damaged (CSD) melanoma accounts for 95% of cutaneous melanomas in the United States and other Western countries. In contrast, acral lentiginous melanoma (ALM) (about 50%) and mucosal melanoma (MM) (about 20%) are the two most common subpopulations of melanoma in Asian populations, as disclosed by Chi, Z., et al in BMC Cancer (2011), 11: 85. Furney, S.J., et al disclosed in Pigment Cell Melanoma Res (2014), 27: 835-8 that both ALM and MM are not associated with chronic UV exposure and carry fewer DNA mutations. A retrospective study disclosed by Cho, J. et al in Invest New Drugs (2016), 34: 677-84 showed that immunotherapy is less effective in treating ALM and MM than in CSD melanoma. Therefore, there is nondeterminacy about the efficacy of immunotherapy, such as anti-PD-1 antibody, in treating melanoma, especially mucosal melanoma and acral melanoma, which are frequently seen in Asian populations, and how to further improve the therapeutic effect of immunotherapy is a technical problem that needs to be solved urgently in the field. In addition, although immune checkpoint inhibitor therapies have made significant improvements in the treatment of metastatic melanoma over the past decade, most of them have focused on cutaneous melanoma (also called non-acral melanoma) of Caucasians, with very limited research in Asian and African populations, leaving a great gap in the treatment of non-acral melanoma and primary melanoma of those two populations that urgently needs to be addressed.

### SUMMARY

In one aspect, the present invention provides a method for treating a patient with melanoma, which comprises administering to the patient a therapeutically effective amount of an anti-PD-1 antibody.

In a second aspect, the present invention provides use of the anti-PD-1 antibody in the preparation of a medicament for treating a patient with melanoma.

By way of one or more embodiments, the melanoma is advanced or metastatic melanoma.

By way of one or more embodiments, the patient with melanoma has a *BRAF* mutation.

By way of one or more embodiments, the patient with melanoma is selected from a patient with acral melanoma, a patient with mucosal melanoma, a patient with non-acral cutaneous melanoma, and a patient with melanoma with indeterminate primary site.

By way of one or more embodiments, the patient with melanoma is a patient with non-acral melanoma.

By way of one or more embodiments, the patient with melanoma is a patient with melanoma with indeterminate primary site.

By way of one or more embodiments, the patient with melanoma has positive PD-L1 expression tested in cancer tissue or section. In one or more embodiments, the patient with melanoma has a tumor mutation burden (TMB) of ≥ 3.6 Muts/Mb in a cancer biopsy sample or a matched peripheral blood sample. In one or more embodiments, the patient with melanoma has both positive PD-L1 expression tested in cancer tissue or section and a tumor mutation burden (TMB) of ≥ 3.6 Muts/Mb in a cancer biopsy sample or a matched peripheral blood sample.

In a third aspect, the present invention provides a method for treating a patient with melanoma, which comprises administering to the patient a therapeutically effective amount of a CDK4/6 inhibitor alone or in combination with a therapeutically effective amount of an anti-PD-1 antibody.

By way of one or more embodiments, the patient with melanoma has a *CDK4* or *CCDN1* gene amplification.

In a fourth aspect, the present invention provides a kit for predicting the therapeutic effect of an anti-PD-1 antibody administered to an individual with a tumor on the tumor, which comprises: (a) an agent for detecting a *BRAF* mutation in tumor tissue or peripheral blood of the individual; and (b) instructions for using the agent of (a) to test the therapeutic effect of the anti-PD-1 antibody administered to the individual.

In a fifth aspect, the present invention provides a kit for predicting the therapeutic effect of an anti-PD-1 antibody administered to an individual with a tumor on the tumor, which comprises: (a) an agent for detecting a *CDK4*/*CCND1* gene mutation or amplification in tumor tissue or peripheral blood of the individual; and (b) instructions for using the agent of (a) to test the therapeutic effect of the CDK4/6 inhibitor administered alone or in combination with the anti-PD-1 antibody to the individual.

In a sixth aspect, the present invention provides a kit for predicting the therapeutic effect of an anti-PD-1 antibody administered to an individual with a tumor on the tumor, which comprises: (a) an agent for detecting an *NRAS* gene mutation in tumor tissue or peripheral blood of the individual; and (b) instructions for using the agent of (a) to test the therapeutic effect of the anti-PD-1 antibody administered to the individual.

In the use, method and kit described herein, the anti-PD-1 antibody is a monoclonal antibody or an antigen-binding fragment thereof. In certain embodiments, the anti-PD-1 antibody can specifically bind to PD-1 and block the binding of PD-L1 or PD-L2 to PD-1. In certain embodiments, the anti-PD-1 antibody specifically binds to PD-L1 or/and PD-L2 and blocks the binding of PD-L1 and/or PD-L2 to PD-1.

In one or more embodiments, the anti-PD-1 antibody is an antibody comprising at least one complementarity determining region (CDR), wherein the complementarity determining region (CDR) comprises an amino acid sequence selected from SEQ ID NO: 1. 2, 3, 4, 5 and 6.

In one or more embodiments, the anti-PD-1 antibody is an antibody comprising complementarity determining regions (CDRs), wherein light chain complementarity determining regions (LCDRs) comprise amino acid sequences set forth in SEQ ID NOs: 1, 2 and 3, and heavy chain complementarity determining regions (HCDRs) comprise amino acid sequences set forth in SEQ ID NOs: 4. 5 and 6.

In one or more embodiments, the anti-PD-1 antibody comprises a light chain variable region (VL) and a heavy chain variable region (VH), wherein the VL comprises an amino acid sequence set forth in SEQ ID NO: 7, and the VH comprises an amino acid sequence set forth in SEQ ID NO: 8.

In one or more embodiments, the anti-PD-1 antibody is an anti-PD-1 antibody comprising a light chain and a heavy chain, wherein the light chain comprises an amino acid sequence set forth in SEQ ID NO: 9, and the heavy chain comprises an amino acid sequence set forth in SEQ ID NO: 10.

In one or more embodiments, the anti-PD-1 antibody is selected from one or more of nivolumab, pembrolizumab, toriplalimab, sintilimab, camrelizumab, tislelizumab and cemiplimab.

In one or more embodiments, the anti-PD-1 antibody or the antigen-binding fragment thereof is administered at a single dose of about 0.1 mg/kg body weight to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 120 mg to about 480 mg, e.g., a fixed dose of about 120 mg, 240 mg, 360 mg or 480 mg.

In one or more embodiments, the anti-PD-1 antibody or the antigen-binding fragment thereof is administered at a frequency of about once every week, once every two weeks, once every three weeks, once every four weeks or once a month, preferably once every two weeks.

In one or more embodiments, the anti-PD-1 antibody or the antigen-binding fragment thereof is administered at a single dose of 1 mg/kg body weight, 3 mg/kg body weight or 10 mg/kg body weight, or of a fixed dose of 240 mg or 480 mg once every two weeks.

In one or more embodiments, the anti-PD-1 antibody or the antigen-binding fragment thereof is administered parentally, e.g., by intravenous infusion, in a liquid dosage form, e.g., an injection.

In one or more embodiments, the anti-PD-1 antibody or the antigen-binding fragment thereof can be administered in cycles of one week, two weeks, three weeks, one month, two months, three months, four months, five months, half a year or longer; optionally, the cycles each can be identical or different, and at identical or different intervals.

In one or more embodiments, the CDK4/6 inhibitor is selected from palbociclib, ribociclib and abemaciclib.

In the use, method and kit described herein, the individual is a human.

In another one or more embodiments of the use, method and kit described herein, the subject has melanoma and has not previously received an anti-PD-1 or anti-PD-L1 immunotherapy. In a preferred embodiment, the cancer is advanced melanoma. In a preferred embodiment, the cancer is metastatic melanoma.

In a seventh aspect, the present invention provides a method for predicting the therapeutic effect of an anti-PD-1 antibody on a patient with a tumor, which comprises detecting a biomarker in tumor tissue or peripheral blood of the patient prior to treatment, wherein the biomarker is a *BRAF* gene mutation, wherein the presence of the *BRAF* gene mutation indicates that the patient with the tumor is suitable for treatment with the anti-PD-1 antibody.

In an eighth aspect, the present invention provides a method for predicting the therapeutic effect of an anti-PD-1 antibody on a patient with a tumor, which comprises detecting a biomarker in tumor tissue of the patient, wherein the biomarker is a *CDK4* or *CCND1* gene amplification. In one or more embodiments, the presence of the *CDK4* or *CCND1* gene amplification indicates that the patient with the tumor is suitable for treatment with the anti-PD-1 antibody in combination with the CDK4/6 inhibitor. In another one or more embodiments, the presence of the *CDK4* or *CCND1* gene amplification indicates that the patient with the tumor is suitable for treatment with the CDK4/6 inhibitor.

In a ninth aspect, the present invention provides a method for predicting the therapeutic effect of an anti-PD-1 antibody on a patient with a tumor, which comprises detecting a biomarker in tumor tissue or peripheral blood of the patient prior to treatment, wherein the biomarker is an *NRAS* gene mutation. In one or more embodiments, the presence of the *NRAS* gene mutation indicates that the therapeutic effect of the anti-PD-1 antibody alone is undesirable or the patient with the tumor is not suitable for treatment with the anti-PD-1 antibody alone.

In the use, method and kit described herein, the tumor is a solid tumor. In one or more embodiments, the tumor is melanoma.

### BRIEF DESCRIPTION OF THE DRAWINGS

FIG. 1: a flowchart of a phase II study on a standard treatment for locally advanced or metastatic melanoma with toripalimab.
FIG. 2: evaluation of clinical responses according to RECIST v1.1. A: maximum change in tumor size from baseline for patients with at least one post-treatment imaging evaluation (n = 119). The length of the bar represents the maximum decrease or minimum increase of the target lesion; B: change in tumor burden in the individual over time from baseline (n = 119); C: the extent of exposure and duration of reaction of the confirmed responders (n = 22).
FIG. 3: progression-free survival PFS (A) and overall survival OS (B) of patients with melanoma in this study.
FIG. 4: overall survival OS (A) of the response subgroup and overall survival OS (B) of the melanoma subgroup in this study.
FIG. 5: progression-free survival PFS of patients with melanoma subtypes in this study.

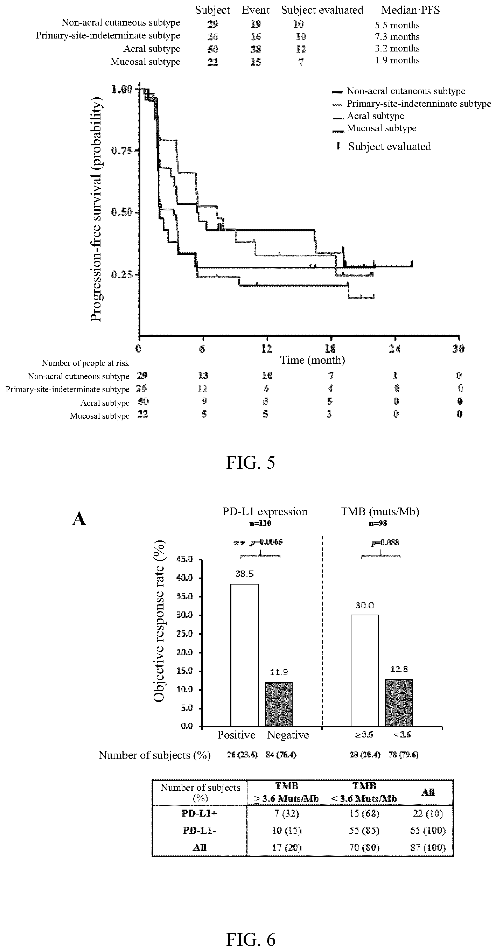

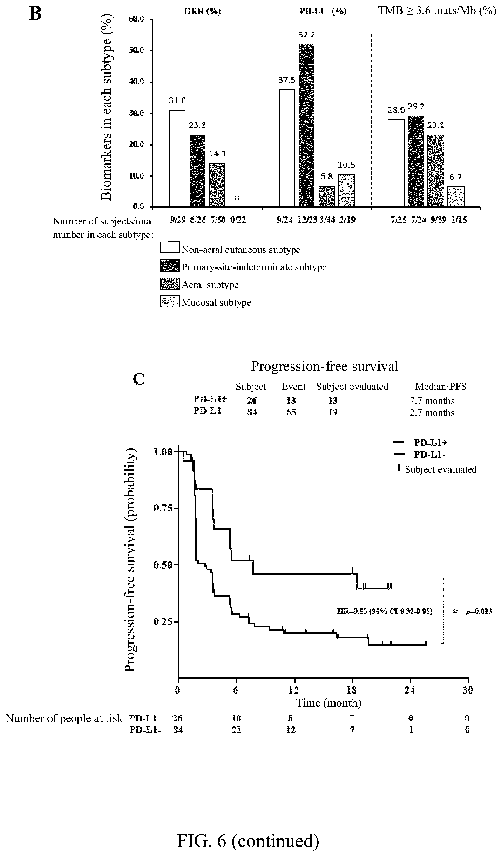

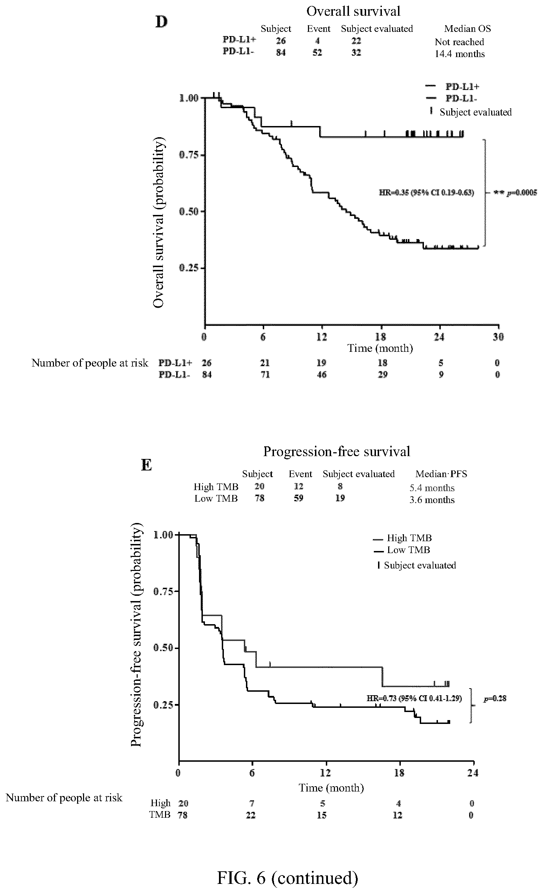

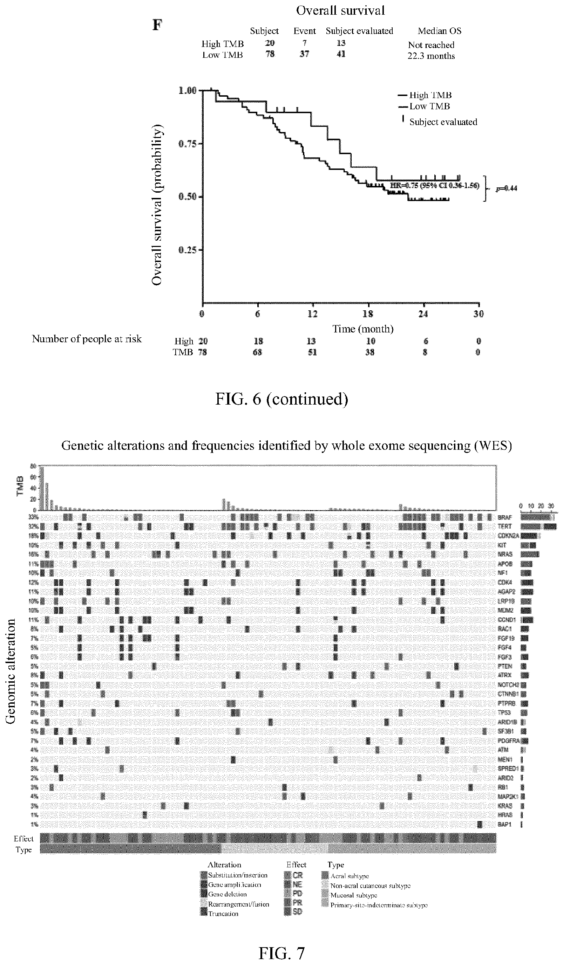

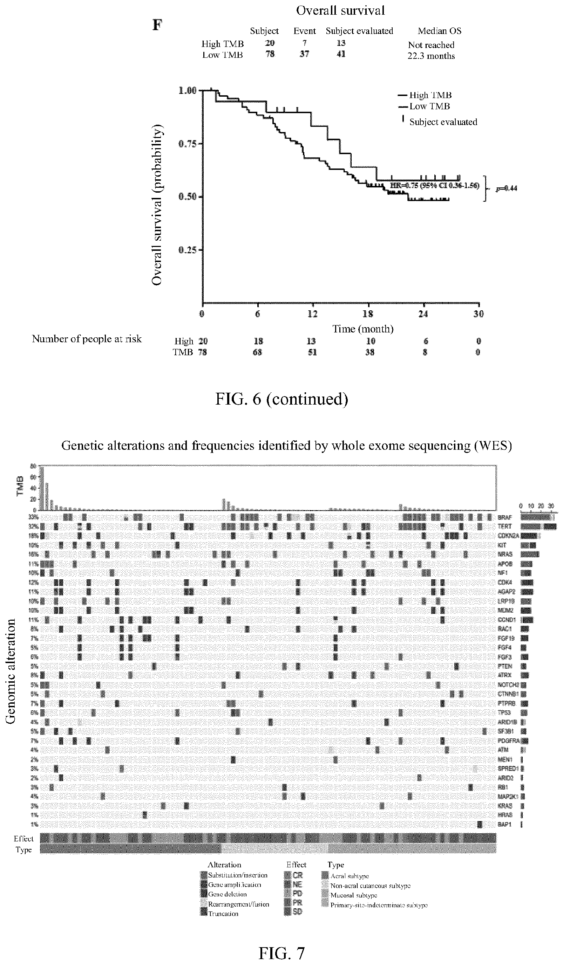

FIG. 6: relationship between clinical response and tumor PD-L1 expression and TMB. A: PD-L1 positive status is defined as the presence of any tumor cells or immune cells with membrane staining intensity ≥ 1% by SP142 IHC staining; TMB is calculated by whole exon sequencing of somatic mutations within the coding region, with 3.6 Muts/Mb as a cut-off value; B: percentage of PD-L1⁺ status or TMB ≥ 3.6 Muts/Mb in the melanoma subgroup; C: progression-free survival PFS of PD-L1⁺ and PD-L1⁻ patients; D: overall survival OS of PD-L1⁺ and PD-L1⁻ patients; E: progression-free survival PFS of patients with TMB ≥ 3.6 Muts/Mb and TMB < 3.6 Muts/Mb; F: overall survival OS of patients with TMB ≥ 3.6 Muts/Mb and TMB < 3.6 Muts/Mb.
FIG. 7: whole exome sequencing (WES) of gene variation and frequency in 98 patients.
FIG. 8: relationship between clinical response and signature scores for IFN-□-related genomes, inflammation-related genomes, and angiogenesis-related genomes.

### DETAILED DESCRIPTION

The present invention relates to a method for treating a tumor. The method of the present invention comprises administering to a patient in need an anti-PD-1 antibody or an antigen-binding fragment thereof alone; or comprises administering to a patient in need an anti-PD-1 antibody in combination with an additional anti-cancer agent. The present invention also relates to a method for predicting the therapeutic effect of an anti-PD-1 antibody on a patient with cancer, preferably a patient with melanoma using a biomarker.

### Terminology

In order to facilitate the understanding of the present invention, some technical and scientific terms are specifically defined below. Unless otherwise specifically defined herein, all technical and scientific terms used herein have the same meaning as commonly understood by those of ordinary skill in the art to which the present invention belongs.

"Administering", "giving" and "treating" refers to introducing a composition comprising a therapeutic agent into a subject using any one of a variety of methods or delivery systems known to those skilled in the art. Routes of administration of the anti-PD-1 antibody include intravenous, intramuscular, subcutaneous, intraperitoneal, spinal or other parenteral routes of administration, such as injection or infusion. "Parenteral administration" refers to modes of administration apart from enteral or local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion, and *in vivo* electroporation.

An "adverse event" (AE) described herein is any adverse and often unintended or undesirable sign, symptom, or disease associated with the use of medical treatment. For example, an adverse event may be associated with the activation of the immune system or the amplification of immune system cells in response to treatment. The medical treatment may have one or more related AEs, and the AEs each may have identical or different severity levels.

"Tumor burden" refers to the total amount of tumor mass distributed throughout the body. Tumor burden refers to the total number of cancer cells or the total size of the tumor throughout the body. Tumor burden can be determined by a variety of methods known in the art, such as measuring the size of a tumor using calipers after the tumor is removed from a subject, or using imaging techniques (e.g., ultrasound, bone scanning, computed tomography (CT), or magnetic resonance imaging (MRI) scanning) when the tumor is *in vivo.*

The term "tumor size" refers to the total size of a tumor, which can be measured as the length and width of the tumor. Tumor size can be determined by a variety of methods known in the art, such as measuring the size of a tumor using calipers after the tumor is removed from a subject, or using imaging techniques (e.g., bone scanning, ultrasound, CT, or MRI scanning) when the tumor is *in vivo.*

The terms "subject" and "individual" include any organism, preferably an animal, more preferably a mammal (such as rat, mouse, dog, cat and rabbit), and most preferably a human. The terms "subject" and "patient" are used interchangeably herein.

An "antibody" described herein refers to any form of antibody that achieves a desired biological or binding activity. Therefore, it is used in the broadest sense, but is not limited to, monoclonal antibodies, polyclonal antibodies, multispecific antibodies, humanized full-length human antibodies, chimeric antibodies and camelid single-domain antibodies. The "antibody" specifically binds to an antigen and comprises at least two heavy (H) chains and two light (L) chains interconnected by disulfide bonds. Each heavy chain comprises a heavy chain variable region (VH) and a heavy chain constant region comprising three constant domains CH1, CH2 and CH3. Each light chain comprises a light chain variable region (VL) and a light chain constant region comprising one constant domain CL. The VH and VL regions can be further divided into hypervariable regions termed complementarity determining regions (CDRs), which are scattered over more conserved regions termed framework regions (FRs). Generally, both light and heavy chain variable domains comprise FR1, CDR1, FR2, CDR2, FR3, CDR3 and FR4 from N-terminus to C-terminus. Amino acids are typically assigned to each domain according to the following definitions: Sequences of Proteins of Immunological Interest, Kabat et al.; National Institutes of Health, Bethesda, Md.; 5th edition; NIH publication No. 91-3242 (1991): Kabat (1978) Adv. Prot. Chem. 32:1-75; Kabat et al., (1977) J. Biol. Chem. 252:6609-6616; Chothia et al., (1987) J Mol. Biol. 196:901-917 or Chothia et al., (1989) Nature 341:878-883.

The carboxyl-terminal portion of the heavy chain can define a constant region primarily responsible for effector function. Human light chains are generally classified as κ and λ chains. Human heavy chains are generally classified as µ, δ, γ, α or ε chains, and isotypes of the antibody are defined as IgM, IgD, IgG, IgA and IgE, respectively. IgG subclass is well known to those skilled in the art and includes, but is not limited to, IgG1, IgG2, IgG3 and IgG4.

The term "antibody" includes: naturally occurring and non-naturally occurring Abs; monoclonal and polyclonal Abs; chimeric and humanized Abs; human or non-human Abs; fully synthetic Abs; and single chain Abs. Non-human Abs can be humanized by recombinant methods to reduce their immunogenicity in humans.

Unless otherwise specifically indicated, an "antibody fragment" or "antigen-binding fragment" described herein refers to an antigen-binding fragment of an antibody, i.e., an antibody fragment that retains the ability of a full-length antibody to specifically bind to an antigen, e.g., a fragment that retains one or more CDR regions. Examples of an antigen-binding fragment include, but are not limited to, Fab, Fab', F(ab')₂, and Fv fragments; a diabody; a linear antibody; a single-chain antibody molecule; and a nanoantibody and a multispecific antibody formed from fragments of the antibody.

A "chimeric antibody" refers to an antibody and a fragment thereof in which a portion of the heavy and/or light chain is identical with or homologous to corresponding sequences of an antibody derived from a particular species (e.g., human) or belonging to a particular antibody class or subclass, while the remainder of the chain is identical with or homologous to corresponding sequences of an antibody derived from another species (e.g., mouse) or belonging to another antibody class or subclass, so long as they exhibit the desired biological activity.

A "human antibody" refers to an antibody that comprises only human immunoglobulin sequences. A human antibody may contain a murine carbohydrate chain if it is produced in mice, mouse cells, or hybridomas derived from mouse cells. Similarly, "mouse antibody" or "rat antibody" refers to an antibody that comprises only mouse or rat immunoglobulin sequences, respectively.

A "humanized antibody" refers to an antibody form containing sequences from both non-human (e.g., murine) and human antibodies. Such antibodies contain minimal sequences derived from non-human immunoglobulins. Typically, a humanized antibody will comprise substantially all of at least one and typically two variable domains, in which all or substantially all of the hypervariable loops correspond to those of a non-human immunoglobulin and all or substantially all of the FR regions are those of a human immunoglobulin. The humanized antibody optionally also comprises at least one portion of an immunoglobulin constant region (Fc), typically a human immunoglobulin constant region.

The term "melanoma" refers to a malignant tumor derived from melanocytes, which is usually found in the skin, as well as in mucous membrane, ocular choroid and other sites. Melanoma is the most malignant tumor species among skin tumors and is prone to distant metastasis. Melanoma is classified as four subtypes, including acral melanoma, mucosal melanoma, cutaneous melanoma and melanoma with indeterminate primary site. Among those, the cutaneous melanoma is further classified as chronic sun-damaged (CSD) melanoma and non-chronic sun-damaged (non-CSD) melanoma, and CSD and non-CSD melanomas are collectively called sun-damaged melanoma. It is disclosed in McLaughlin et al., Cancer, 2005, Mar 1,103(5): 1000-1007; chi Z, et al., BMC Cancer, 2011; 11:85 that acral melanoma and mucosal melanoma are the most prominent subtypes in Asians, and their carcinogenesis is not caused by DNA mutations resulting from ultraviolet radiation. Compared to the sun-damaged melanoma, acral melanoma and mucosal melanoma contain only a few DNA mutations. In the United States, 95% of melanomas are sun-damaged melanoma, while in Asia, especially in China, more than 70% of melanomas are acral melanoma and mucosal melanoma, with mucosal melanomas accounting for more than 50%. It is disclosed by Cho, J, et al in Invest New Drugs, 34: 677-684 that immunotherapy is less effective in treating acral melanoma and mucosal melanomas than in sun-damaged melanoma.

The term "immunotherapy" refers to the treatment of a subject with a disease or at risk of infection or disease recurrence by a method that includes inducing, enhancing, suppressing or otherwise modifying an immune response. The "treatment" or "therapy" of a subject refers to any type of intervention or process performed on the subject, or the administration of an active agent to the subject, with the purpose of reversing, alleviating, ameliorating, slowing or preventing the onset, progression, severity, or recurrence of symptoms, complications or conditions, or biochemical indicators associated with the disease.

A "programmed death receptor-1 (PD-1)" refers to an immunosuppressive receptor belonging to the CD28 family. PD-1 is expressed primarily on previously activated T cells *in vivo* and binds to two ligands, PD-L1 and PD-L2. The term "PD-1" used herein includes human PD-1 (hPD-1), variants, isotypes, and species homologs of hPD-1, and analogs having at least one common epitope with hPD-1.

A "therapeutically effective amount" or "therapeutically effective dose" of a medicament or therapeutic agent is any amount of the medicament that, when used alone or in combination with an additional therapeutic agent, protects a subject from the onset of a disease or promotes the regression of a disease as evidenced by a decrease in the severity of disease symptoms, an increase in the frequency and duration of disease symptom-free phase, or the prevention of injury or disability resulting from the affliction of the disease. The ability of a therapeutic agent to promote the regression of a disease can be assessed using a variety of methods known to those skilled in the art, such as in human subjects during clinical trials, in animal model systems that predict human efficacy, or by determining the activity of the agent in an *in vitro* assay.

A therapeutically effective amount of a medicament includes a "prophylactically effective amount" which is any amount of a medicament that, when administered alone or in combination with an anti-neoplastic agent, inhibits the development or recurrence of cancer to a subject at risk of developing cancer or a subject having cancer recurrence.

A "biotherapeutic agent" refers to a biomolecule, such as an antibody or fusion protein, that blocks ligand/receptor signaling in any biological pathway that supports tumor maintenance and/or growth or inhibits an anti-tumor immune response.

Unless otherwise specifically indicated, "CDR" used herein refers to a complementarity determining region of the immunoglobulin variable region defined using the Kabat numbering system.

A "therapeutic anti-PD-1 monoclonal antibody" refers to an antibody that specifically binds to the mature form of a specific PD-1 expressed on the surface of certain mammalian cells. Mature PD-1 does not have a secretory leader sequence, or leader peptide. The terms "PD-1" and "mature PD-1" are used interchangeably herein and are to be understood as the same molecule unless otherwise specifically defined, or clearly seen from the context.

A therapeutic anti-human PD-1 antibody or anti-hPD-1 antibody described herein refers to a monoclonal antibody that specifically binds to mature human PD-1.

A "framework region" or "FR" described herein refers to the immunoglobulin variable region excluding CDR regions.

An "isolated antibody or antigen-binding fragment thereof" refers to a molecule that is in a purified state, and in this case, is substantially free of other biomolecules, such as nucleic acids, proteins, lipids, carbohydrates, or other materials (such as cell debris or growth medium).

A "patient" or "subject" refers to any single subject in need of a medical procedure or participating in a clinical trial, epidemiological study, or serving as a control, and is generally a mammal, including a human and other mammals, such as horses, cows, dogs or cats.

The "RECIST 1.1 efficacy criteria" described herein refers to the definition of target injury and non-target injury described in Eisenhauver et al., E.A. et al., Eur. J Cancer 45:228-247(2009) in the context of the measured background. RECIST 1.1 efficacy criteria is the most commonly used criteria for efficacy evaluation of solid tumors prior to immunotherapy. However, with the advent of the immunotherapy, many difficulties that had not previously appeared in tumor evaluation have emerged. Therefore, based on the newly emerging phenomenon brought by immunotherapy itself, the RECIST working group revised the existing "RECIST V.1.1" and proposed a new judgment criterion in 2016, namely the "irRECIST criteria" described herein, aiming at better evaluating the efficacy of immunotherapeutic drugs.

The term "ECOG" score standard is an indicator of general health status and tolerance to treatment of patients from their physical strength. ECOG score standard for the physical strength is 0 points, 1 point, 2 points, 3 points, 4 points and 5 points. A score of 0 means that the motility is completely normal and has no difference from the motility before onset of disease. A score of 1 means that the person is free to walk and can engage in light physical activities, including general housework or office work, but not in heavy physical activities.

A "sustained response" refers to a sustained therapeutic effect following cessation of treatment with a therapeutic agent or combination therapy described herein. In some embodiments, the sustained response has a duration that is at least the same as the duration of treatment or at least 1.5, 2.0, 2.5 or 3 times the duration of the treatment.

A "tissue section" refers to a single portion or piece of a tissue sample, such as a tissue slice cut from a sample of normal tissue or a tumor.

"Treating" cancer described herein refers to administering a treatment regimen described herein (e.g., administration of an anti-PD-1 antibody or a combination therapy of an anti-PD-1 antibody with a CDK4/6 inhibitor) to a subject with or diagnosed with cancer to achieve at least one positive therapeutic effect (e.g., a decrease in cancer cell number, a decrease in tumor volume, a reduction in the rate of cancer cell infiltration into peripheral organs, or a reduction in the rate of tumor metastasis or tumor growth). Positive therapeutic effects in cancer can be measured in a variety of ways (see W. A. Weber, J. Nucl. Med., 50:1S-10S (2009)). For example, T/C ≤ 42% for tumor growth inhibition is the minimum level of anti-tumor activity according to the NCI criteria. It is considered that T/C (%) = median treated tumor volume/median control tumor volume × 100. In some embodiments, the therapeutic effect achieved by the combination of the present invention is any one of PR, CR, OR, PFS, DFS and OS. PFS (also called "time to tumor progression") refers to the length of time during and after treatment for which cancer does not grow, and includes the amount of time a patient experiences CR or PR and the amount of time a patient experiences SD. DFS refers to the length of time during and after treatment for which a patient remains disease-free. OS refers to an extension of life expectancy compared to an initial or untreated individual or a patient. In some embodiments, the response to the combination of the present invention is any one of PR, CR, PFS, DFS, OR or OS, assessed using RECIST 1.1 efficacy criteria. The treatment regimen of the combination of the present invention effective in treating a patient with cancer may vary depending upon a variety of factors such as the disease state, age, weight of the patient and the ability of the therapy to elicit an anti-cancer response in the subject. Embodiments of the present invention may not achieve an effective positive therapeutic effect in each subject, but should be effective and achieve a positive therapeutic effect in a statistically significant number of subjects.

The terms "mode of administration" and "dosing regimen" are used interchangeably and refer to the dosage and time of use of each therapeutic agent in the combination of the present invention.

The term "immunohistochemistry (IHC)" refers to a method for determining antigens (polypeptides and proteins) in tissue cells by developing chromogenic agents (fluoresceins, enzymes, metal ions, isotopes) that label antibodies through chemical reaction based on the principle that antigens specifically bind to antibodies, and performing localized, qualitative and relatively quantitative studies on those antigens. In some embodiments of the present invention, a tumor tissue sample from a subject is tested for PD-L1 prior to treatment with an anti-PD-1 antibody by staining the anti-human PD-L1 antibody SP142 from Roche (Cat No: M4422). In some embodiments, the presence of tumor cells with membrane staining ≥ 1% is defined as PD-L1 positive.

In the following paragraphs, various aspects of the present invention are further described in detail.

### Anti-PD-1 antibody

An "anti-PD-1 antibody" used herein refers to any chemical compound or biomolecule that binds to the PD-1 receptor, blocks the binding of PD-L1 expressed on cancer cells to PD-1 expressed on immune cells (T, B and NK cells), and preferably blocks the binding of PD-L2 expressed on cancer cells to PD-1 expressed on immune cells. Alternative nouns or synonyms for PD-1 and its ligands include: PDCD1, PD1, CD279 and SLEB2 for PD-1; PDCD1L1, PDL1, B7-H1, B7H1, B7-4, CD274 and B7-H for PD-L1; and PDCD1L2, PDL2, B7-DC and CD273 for PD-L2. In any of the therapy, medicament and use described herein for treating a human individual, the anti-PD-1 antibody blocks the binding of human PD-L1 to human PD-1, and preferably blocks the binding of both human PD-L1 and PD-L2 to human PD-1. The amino acid sequence of human PD-1 can be found at NCBI locus number: NP_005009. The amino acid sequences of human PD-L1 and PD-L2 can be found at NCBI locus numbers: NP_054862 and NP_079515.

As used herein, unless otherwise indicated or described, when referring to the term "anti-PD-1 antibody", it includes antigen-binding fragments of the antibody.

The anti-PD-1 antibody suitable for any of the use, therapy, medicament and kit described herein has an immunosuppressive effect achieved by binding to PD-1 with high specificity and high affinity, blocking the binding of PD-L1/2 to PD-1 and inhibiting PD-1 signal transduction. In any of the use, therapy, medicament and kit disclosed herein, the anti-PD-1 antibody includes the full-length antibody itself, as well as an antigen-binding portion or fragment that binds to the PD-1 receptor and exhibits functional properties similar to an intact Ab in inhibiting ligand binding and upregulating the immune system. In some embodiments, the anti-PD-1 antibody or the antigen-binding fragment thereof is an anti-PD-1 antibody or an antigen-binding fragment thereof that cross-competes for binding to human PD-1 with toripalimab. In other embodiments, the anti-PD-1 antibody or the antigen-binding fragment thereof is a chimeric, humanized or human Ab or an antigen-binding fragment thereof. In certain embodiments for treating a human individual, the Ab is a humanized Ab.

In some embodiments, the anti-PD-1 antibody that can be used in any of the use, therapy, medicament and kit described herein includes a monoclonal antibody (mAb) or an antigen-binding fragment thereof that specifically binds to PD-1, and preferably specifically binds to human PD-1. The mAb may be a human antibody, a humanized antibody, or a chimeric antibody, and may include a human constant region. In some embodiments, the constant region is selected from human IgG1, IgG2, IgG3 and IgG4 constant regions; preferably, the anti-PD-1 antibody or the antigen-binding fragment thereof suitable for use in any of the use, therapy, medicament and kit described herein comprises a heavy chain constant region of human IgG1 or IgG4 isotype, more preferably a human IgG4 constant region. In some embodiments, the sequence of the IgG4 heavy chain constant region of the anti-PD-1 antibody or the antigen-binding fragment thereof comprises the S228P mutation that replaces a serine residue in the hinge region with a proline residue that is typically present at the corresponding position of an antibody of IgG1 isotype.

Preferably, in any one of the embodiments of the use, therapy, medicament and kit described herein, the anti-PD-1 antibody is a monoclonal antibody or an antigen-binding fragment thereof comprising light chain CDRs and heavy chain CDRs, wherein the light chain CDRs comprise amino acid sequences set forth in SEQ ID NOs: 1, 2 and 3, and the heavy chain CDRs comprise amino acid sequences set forth in SEQ ID NOs: 4, 5 and 6.

More preferably, in any one of the embodiments of the use, therapy, medicament and kit described herein, the anti-PD-1 antibody is a monoclonal antibody that specifically binds to human PD-1 and comprises: (a) a light chain variable region comprising an amino acid sequence set forth in SEQ ID NO: 7, and (b) a heavy chain variable region comprising an amino acid sequence set forth in SEQ ID NO: 8.

Further preferably, in any one of the embodiments of the use, therapy, medicament and kit described herein, the anti-PD-1 antibody is a monoclonal antibody that specifically binds to human PD-1 and comprises: (a) a light chain set forth in SEQ ID NO: 9, and (b) a heavy chain set forth in SEQ ID NO: 10.

Table A below provides the amino acid sequence numbering of the light chain CDRs and heavy chain CDRs of an exemplary anti-PD-1 antibody mAb for use in the use, therapy, medicament and kit described herein:

**Table A. Light and heavy chain CDRs (Kabat) of an exemplary anti-human PD-1 antibody**

| | |
|---|---|
| LCDR1 | SEQ ID NO: 1 |
| LCDR2 | SEQ ID NO: 2 |
| LCDR3 | SEQ ID NO: 3 |
| HCDR1 | SEQ ID NO: 4 |
| HCDR2 | SEQ ID NO: 5 |
| HCDR3 | SEQ ID NO: 6 |

An example of anti-PD-1 antibodies that bind to human PD-1 and can be used in the use, therapy, medicament and kit described herein is described in WO2014206107. Human PD-1 mAbs that can be used as anti-PD-1 antibodies in the use, therapy, medicament and kit described herein include any one of the anti-PD-1 antibodies described in WO2014206107, including toripalimab (a humanized IgG4 mAb having the structure described in WHO Drug Information; 32(2):372-373 (2018) and comprising light and heavy chain amino acid sequences set forth in SEQ ID NOs: 9 and 10). In a preferred embodiment, the anti-PD-1 antibody that can be used in any one of the use, therapy, medicament and kit described herein is selected from humanized antibodies 38, 39, 41 and 48 described in WO2014206107. In a particularly preferred embodiment, the anti-PD-1 antibody that can be used in any one of the use, therapy, medicament and kit described herein is toripalimab.

The anti-PD-1 antibody that can used in any of the use, therapy, medicament and kit described herein also includes nivolumab and pembrolizumab that have been approved by the FDA.

In certain embodiments, the anti-PD-1 antibody that can be used in any one of the use, therapy, medicament and kit described herein also includes an anti-PD-L1 monoclonal antibody that specifically binds to PD-L1 to block the binding of PD-L1 to PD-1, such as nivolumab, pembrolizumab, toripalimab, sintilimab, camrelizumab, tislelizumab and cemiplimab.

"PD-L1" expression or "PD-L2" expression described herein refers to any detectable expression level of a specific PD-L protein on the surface of a cell or a specific PD-L mRNA within a cell or tissue. PD-L protein expression can be detected in IHC analysis of tumor tissue sections or by flow cytometry using diagnostic PD-L antibodies. Alternatively, PD-L protein expression of tumor cells can be detected by PET imaging using a binding agent that specifically binds to a desired PD-L target (such as PD-L1 or PD-L2).

Methods for quantifying PD-L1 protein expression in IHC analysis of tumor tissue sections are found in, but are not limited to, Thompson, R. H. et al., PNAS 101(49):17174-17179 (2004); Taube, J. M. et al., Sci Transl Med 4, 127ra37 (2012); and Topalian, S. L. et al., New Eng. J. Med. 366(26): 2443-2454 (2012), and the like.

In one method, a simple binary endpoint of positive or negative PD-L1 expression is adopted, where the positive expression is defined by the percentage of tumor cells showing histological evidence of cell surface membrane staining. The case where tumor cells on a tumor tissue section account for at least 1% of the total tumor cells is defined as positive PD-L1 expression.

In another method, PD-L1 expression in the tumor tissue section is quantified in tumor cells as well as in infiltrating immune cells. The percentage of tumor cells and infiltrating immune cells exhibiting membrane staining are quantified individually as < 1%, 1% to 50%, and subsequent 50% to 100%. For tumor cells, the PD-L1 expression is counted as negative if the score is < 1%, and positive if the score is ≥ 1%.

In some embodiments, the expression level of PD-L1 by malignant cells and/or by infiltrating immune cells within the tumor is determined to be "overexpressed" or "elevated" based on comparison with the expression level of PD-L1 by an appropriate control. For example, the protein or mRNA expression level of control PD-L1 can be a quantified level in nonmalignant cells of the same type or in sections from matched normal tissue.

### CDK4/6 inhibitor

The term "CDKs (cyclin-dependent kinases)" used herein is a group of serine/threonine protein kinases. The CDKs drive the cell cycle by phosphorylation of serine/threonine proteins through synergistic interaction with cyclin and are important factors in cell cycle regulation. The CDK family has 8 types, including CDK1-8, each of which binds to cyclin of different types to form a complex that regulates the transition of cells from G1 phase to S phase or G2 phase to M phase and the exiting from M phase.

Currently, the CDK4/6 inhibitors that have been approved by the FDA for marketing mainly include ribociclib, palbociclib, abemaciclib, etc. Ribociclib is used in combination with an aromatase inhibitor as a first-line therapy for HR-positive and HER2-negative postmenopausal female patients with advanced metastatic breast cancer; palbociclib is used in combination with letrozole for treating estrogen receptor (ER)-positive and human epidermal growth factor receptor 2 (HER2)-negative postmenopausal female patients with metastatic breast cancer; abemaciclib is mainly used for treating hormone receptor (HR)-positive and human epidermal growth factor receptor 2 (HER2)-negative adult patients with advanced or metastatic breast cancer who have progressive disease after receiving endocrine therapy.

There are dozens of CDK4/6 inhibitors in clinical research, such as milciclib, trilaciclib, lerociclib, and voruciclib.

In any of the use, therapy, medicament and kit described herein, the CDK4/6 inhibitor includes, but is not limited to, palbociclib.

In any of the use, therapy, medicament and kit described herein, the combination includes, but is not limited to, a combination of toripalimab with palbociclib.

### Disease and Treatment Thereof

The present invention relates to the treatment of cancer. The present invention comprises administering to a patient in need an anti-PD-1 antibody or an antigen-binding fragment thereof alone; or comprises administering to a patient in need an anti-PD-1 antibody in combination with an additional anti-cancer agent.

The term "cancer" used herein refers to a wide variety of diseases characterized by the uncontrolled growth of abnormal cells in the body. Unregulated cell division, growth division and growth lead to the formation of malignant tumors that invade adjacent tissues and can also metastasize to distal parts of the body through the lymphatic system or the blood flow. Examples of cancers suitable for treatment or prevention using the method, medicament and kit of the present invention include, but are not limited to, carcinoma, lymphoma, leukemia, blastoma and sarcoma. More specific examples of cancer include squamous cell cancer, myeloma, small-cell lung cancer, non-small cell lung cancer, glioma, Hodgkin lymphoma, non-Hodgkin lymphoma, acute myeloid leukemia, multiple myeloma, gastrointestinal cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, gastric cancer, bladder cancer, hepatoma, breast cancer, colon cancer, and head and neck cancer.

Preferably, the patient with cancer suitable for the present invention is a patient with cancer who has positive PD-L1 expression, or a patient with cancer who has tumor mutation burden (TMB) of ≥ 3.6 Muts/Mb in peripheral blood or tumor tissue, or a patient with cancer who has both positive PD-L1 expression and tumor mutation burden (TMB) of ≥ 3.6 Muts/Mb in peripheral blood or tumor tissue. In certain embodiments, the patient with cancer suitable for the method of the present invention is preferably a patient with cancer who has a *BRAF* gene mutation detected in tumor tissue.

The term "tumor mutation burden (TMB)" used herein refers to the total number of gene coding errors, base substitutions, gene insertion or deletion errors detected in a somatic cell per million bases. In some embodiments of the present invention, tumor mutation burden (TMB) is estimated by analysis of somatic mutations, including coding base substitutions and the megabase insertions of the panel sequences studied. In the present invention, when a subject has a tumor mutation burden (TMB) of ≥ 3.6 Muts/Mb, it is predicted that the therapeutic effect of an anti-PD-1 antibody alone or an anti-PD-1 antibody in combination with other anti-cancer agents being administered to such a subject can be better than being administered to those with TMB < 3.6 Muts/Mb.

The method for treating cancer of the present invention comprises administering to a subject in need a therapeutically effective amount of an anti-PD-1 antibody, or an anti-PD-1 antibody in combination with a CDK4/6 inhibitor. The anti-PD-1 antibody may be preferably as described in any one of the embodiments herein, more preferably an antibody comprising light chain CDRs with amino acid sequences set forth in SEQ ID NOs: 1, 2 and 3 and heavy chain CDRs with amino acid sequences set forth in SEQ ID NOs: 4, 5 and 6, even more preferably a monoclonal antibody comprising a light chain variable region with an amino acid sequence set forth in SEQ ID NO: 7 and a heavy chain variable region with an amino acid sequence set forth in SEQ ID NO: 8, still more preferably a monoclonal antibody comprising a light chain with an amino acid sequence set forth in SEQ ID NO: 9 and a heavy chain with an amino acid sequence set forth in SEQ ID NO: 10, yet more preferably the humanized antibodies 38, 39, 41 and 48 described in Patent No. WO2014206107, and most preferably toripalimab. The CDK4/6 inhibitor is palbociclib, milciclib, trilaciclib, lerociclib and voruciclib, preferably palbociclib. In a preferred embodiment, the present invention uses a combination of toripalimab with palbociclib for treating cancer.

In a particularly preferred embodiment, the present invention provides a method for treating melanoma, which comprises administering to a patient with melanoma a therapeutically effective amount of toripalimab; preferably, the patient has positive PD-L1 expression, or has tumor mutation burden (TMB) of ≥ 3.6 Muts/Mb in peripheral blood or tumor tissue, or has both positive PD-L1 expression and tumor mutation burden (TMB) of ≥ 3.6 Muts/Mb in peripheral blood or tumor tissue. In certain embodiments, the patient with melanoma is preferably a patient with melanoma who has a *BRAF* gene mutation detected in tumor tissue, more preferably a patient with non-acral melanoma or a patient with primary melanoma. In some embodiments, the melanoma is an acral or/and mucosal melanoma. In another particularly preferred embodiment, the present invention provides a method for treating melanoma, which comprises administering to a patient with melanoma a therapeutically effective amount of toripalimab and palbociclib; preferably, the patient has a *CDK4* or *CCND1* gene amplification detected in peripheral blood or tumor tissue.

When two or more therapeutic agents (i.e., "combination therapy") are administered, each of the therapeutic agents can be administered alone or in a pharmaceutical composition comprising the therapeutic agent and one or more pharmaceutically acceptable carriers, excipients and diluents, according to standard pharmaceutical practice.

Each of the therapeutic agents in the combination therapy of the present invention can be administered simultaneously, concurrently or sequentially in any order. The therapeutic agents in the combination therapy are administered in different dosage forms, such as one medicament being a tablet or a capsule and the other medicament being a sterile liquid, and/or at different dosing times, such as the chemotherapeutic agent being administered at least daily and the biotherapeutic agent being administered infrequently, such as once every week, or every two weeks or every three weeks.

In some embodiments, the CDK4/6 inhibitor is administered prior to the administration of the anti-PD-1 antibody, while in other embodiments, the CDK4/6 inhibitor is administered after the administration of the anti-PD-1 antibody.

In some embodiments, at least one of the therapeutic agents in the combination therapy is administered using the same dosing regimen (dose, frequency, duration of treatment) when the medicaments are used as a monotherapy for treating the same cancer.

Each of the small molecule therapeutic agents in the combination therapy described herein can be administered orally or parenterally (such as by intravenous, intramuscular, intraperitoneal, subcutaneous, rectal, topical, or transdermal routes of administration).

The combination therapy described herein can be administered before or after surgery and can be administered before, during or after radiotherapy.

In some embodiments, the combination therapy described herein is administered to a patient who has not received a previous treatment with a biotherapeutic agent (typically an anti-PD-1 antibody) or a chemotherapeutic agent (typically a CDK4/6 inhibitor). In other embodiments, the combination therapy is administered to a patient who fails to achieve a sustained response after the treatment with a biotherapeutic agent or a chemotherapeutic agent.

The combination therapy described herein can be used in the treatment of tumors found by palpation or by imaging techniques known in the art, such as MRI, ultrasound or CAT scanning.

The combination therapy described herein is preferably administered to a patient with cancer who has positive PD-L1 expression tested or has a TMB of ≥ 3.6 Muts/Mb.

The dosing regimen for the combination therapy of the present invention is selected depending on several factors including, but not limited to, serum or tissue conversion rate, degree of symptoms, immunogenicity, and the accessibility of the target cell, tissue, and organ of the treated individual. Preferably, the dosing regimen is designed to deliver the maximum amount of each therapeutic agent to the patient based on an acceptable degree of side effects. Therefore, the dose and dosing frequency of each of the biotherapeutic agents and chemotherapeutic agents in the combination therapy depends on the specific therapeutic agent, the severity of the cancer being treated and the characteristics of the patient.

The anti-PD-1 antibody and the CDK4/6 inhibitor described herein can be provided as a kit comprising a first container and a second container as well as a package insert.

The first container contains at least one dose of a drug comprising an anti-PD-1 antibody, the second container contains at least one dose of a drug comprising a CDK4/6 inhibitor, and the package insert or label comprises instructions for using the drug to treat cancer. The kit can further comprise other materials that can be used in the administration of a medicament, such as diluents, filter paper, IV bags and threads, needles and syringes. By way of a preferred embodiment, the instructions may state that the medicament is intended for use in the treatment of a patient with cancer who has positive PD-L1 expression as tested by immunohistochemical staining (IHC) analysis.

The therapeutic agent described herein can constitute a pharmaceutical composition, such as a pharmaceutical composition comprising the anti-PD-1 antibody described herein or/and an additional anti-cancer agent other than the anti-PD-1 antibody, and an additional pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. Preferably, the carrier suitable for the composition comprising the anti-PD-1 antibody is suitable for intravenous, intramuscular, subcutaneous, parenteral, spinal, or epidermal administration, such as by injection or infusion, while the carrier suitable for the composition comprising an additional anti-cancer agent is suitable for enteral administration, such as oral administration. The pharmaceutical composition of the present invention can contain one or more pharmaceutically acceptable salts, antioxidants, water, non-aqueous carriers, and/or adjuvants such as preserving agent, wetting agent, emulsifying agent, and dispersing agent.

The dosing regimen is adjusted to provide the optimal desired response, such as the maximum therapeutic response and/or the minimum adverse effect. The dose of the anti-PD-1 antibody, when administered in combination with another anti-cancer agent, can range from about 0.01 mg/kg body weight to about 20 mg/kg body weight, about 0.1 mg/kg body weight to about 10 mg/kg body weight, or can be a fixed dose of 120 mg, 240 mg, 360 mg or 480 mg. For example, the dose can be about 0.1 mg/kg body weight, about 0.3 mg/kg body weight, about 1 mg/kg body weight, about 2 mg/kg body weight, about 3 mg/kg body weight, about 5 mg/kg body weight, or about 10 mg/kg body weight. The dosing regimen is generally designed to achieve an exposure that results in sustained receptor occupancy (RO) based on the typical pharmacokinetics of Ab. A representative dosing regimen may be about once a week, about once every two weeks, about once every three weeks, about once every four weeks, about once a month, or longer. In some embodiments, the anti-PD-1 antibody is administered to an individual about once every two weeks.

The dosing schedule of the additional anti-cancer agent varies for different medicaments. In some embodiments of the present invention, the dosing schedule of the CDK inhibitor varies for different subtypes.

### Use

The present invention also comprises an anti-PD-1 antibody or a combination thereof with a CDK4/6 inhibitor for use in the treatment of a patient with cancer, and use of the anti-PD-1 antibody or the combination thereof with the CDK4/6 inhibitor in the preparation of a medicament for treating a patient with cancer. The patient with cancer or the cancer from which the patient suffers may be as described in any of the preceding embodiments; preferably, the patient with cancer is a patient with cancer who has positive PD-L1 expression, or a patient with cancer who has tumor mutation burden (TMB) of ≥ 3.6 Muts/Mb in peripheral blood or tumor tissue, or a patient with cancer who has both positive PD-L1 expression and tumor mutation burden (TMB) of ≥ 3.6 Muts/Mb in peripheral blood or tumor tissue. In certain embodiments, the patient with cancer is a patient with cancer who has a *BRAF* gene mutation detected in tumor tissue. More preferably, the patient with melanoma has positive PD-L1 expression, or has tumor mutation burden (TMB) of ≥ 3.6 Muts/Mb in peripheral blood or tumor tissue, or has both positive PD-L1 expression and tumor mutation burden (TMB) of ≥ 3.6 Muts/Mb in peripheral blood or tumor tissue. In certain embodiments, the patient with melanoma is more preferably a patient with non-acral melanoma or a patient with melanoma with indeterminate primary site. In some embodiments, the patient with melanoma is a patient with acral melanoma or a patient with mucosal melanoma.

The anti-PD-1 antibody for use in the treatment of cancer may be preferably as described in any one of the embodiments herein, more preferably an antibody comprising light chain CDRs with amino acid sequences set forth in SEQ ID NOs: 1, 2 and 3 and heavy chain CDRs with amino acid sequences set forth in SEQ ID NOs: 4, 5 and 6, even more preferably a monoclonal antibody comprising a light chain variable region with an amino acid sequence set forth in SEQ ID NO: 7 and a heavy chain variable region with an amino acid sequence set forth in SEQ ID NO: 8, still more preferably a monoclonal antibody comprising a light chain with an amino acid sequence set forth in SEQ ID NO: 9 and a heavy chain with an amino acid sequence set forth in SEQ ID NO: 10, yet more preferably the humanized antibodies 38, 39, 41 and 48 described in Patent No. WO2014206107, and most preferably toripalimab. The CDK4/6 inhibitor is preferably palbociclib.

In a preferred embodiment, the present invention relates to toripalimab or a combination of toripalimab with palbociclib for use in the treatment of a patient with cancer. More specifically, the present invention relates to toripalimab or a combination of toripalimab with palbociclib for use in the treatment of a patient with melanoma; more preferably, the patient with melanoma has positive PD-L1 expression, or has tumor mutation burden (TMB) of ≥ 3.6 Muts/Mb in peripheral blood or tumor tissue, or has both positive PD-L1 expression and tumor mutation burden (TMB) of ≥ 3.6 Muts/Mb in peripheral blood or tumor tissue, or has a *BRAF* gene mutation detected in tumor tissue, more preferably the patient with melanoma is a patient with non-acral melanoma or a patient with primary melanoma. In some embodiments, the patient with melanoma is a patient with acral melanoma or a patient with mucosal melanoma.

### Method for Predicting Therapeutic Effect of Anti-PD-1 Antibody on Cancer

Herein, the therapeutic effect of an anti-PD-1 antibody, a CDK4/6 inhibitor, or a combination of both for anti-tumor therapy can be predicted by performing a whole exome sequencing (e.g., using second generation sequencing techniques) on a tumor biopsy sample or a corresponding peripheral blood sample from a patient to identify whether certain genes are mutated or amplified.

"Gene mutation" or "gene alteration" described herein includes gene truncation, gene rearrangement/fusion, gene amplification, gene deletion, and gene substitution/insertion, etc.

The term "gene amplification" used herein refers to a process in which the copy number of a gene encoded by a specific protein is increased selectively while the number of the other genes is not increased proportionally. Under natural conditions, the gene amplification is achieved by excising repeated sequences of a gene from the chromosome and then performing extrachromosomal replication in a plasmid, or by transcribing all the repeated sequences of ribosomal RNA to give RNA transcripts and then transcribing them to give additional copies of the original DNA molecule. In the present invention, gene sequencing analysis is disclosed in some examples.

In some embodiments of the present invention, the subject described herein has certain unique gene mutations, for example, some subjects have a *CDK4* or *CDDN1* gene amplification; some subjects have a *BRAF* gene mutation, particularly, subjects with non-acral melanoma or melanoma with indeterminate primary site have a *BRAF* gene mutation; some subjects with mucosal melanoma have an *NF1* gene mutation; some subjects (including patients with acral melanoma, mucosal melanoma, non-acral (cutaneous) melanoma and melanoma with an unknown primary) have an *NRAS* gene mutation. In some embodiments, the *BRAF* gene mutation is a gene rearrangement/fusion and a gene substitution/insertion. In some embodiments, the *NRAS* gene mutation is a gene substitution/insertion.

In some embodiments of the present invention, the case where the patient has a *BRAF* gene mutation is predictive of a better therapeutic effect of the anti-PD-1 antibody of the present invention on the patient. In some embodiments of the present invention, the case where a patient has a *CDK4* or *CCND1* gene amplification is predictive of a better therapeutic effect of the anti-PD-1 antibody of the present invention in combination with a CDK4/6 inhibitor or a CDK4/6 inhibitor alone on the patient. In some embodiments of the present invention, the case where a patient has an *NRAS* gene mutation is predictive of an undesirable therapeutic effect of the anti-PD-1 antibody of the present invention alone on the patient.

Accordingly, the present invention provides a method for predicting the therapeutic effect of the anti-PD-1 antibody of the present invention, particularly toripalimab, on cancer of an individual, which comprises detecting a biomarker in peripheral blood of the patient prior to the treatment, wherein the biomarker is selected from, but not limited to, *BRAF, NRAS, CDK4* and *CCND1.* The presence of a *BRAF* gene mutation indicates that the subject is suitable for treatment with an anti-PD-1 antibody, the presence of an *NRAS* gene mutation indicates that the subject is not suitable for treatment with an anti-PD-1 antibody, and the presence of a *CDK4* or *CCND1* gene amplification indicates that the subject is suitable for treatment with an anti-PD-1 antibody and a CDK4/6 inhibitor or for treatment with a CDK4/6 inhibitor alone. Preferably, the cancer is melanoma.

The present invention also comprises a method for predicting the therapeutic effect of an anti-PD-1 antibody on a patient with a tumor using the *BRAF* gene or *NRAS* gene. The presence of a *BRAF* gene mutation indicates that the patient with a tumor is suitable for treatment with an anti-PD-1 antibody. The presence of an *NRAS* gene mutation indicates that the patient with a tumor is not suitable for treatment with an anti-PD-1 antibody.

In certain embodiments, the present invention also provides use of a detection agent for a biomarker (particularly a *BRAF* gene, and/or *NRAS* gene, and/or *CDK4* gene, and/or *CCND1* gene) in the preparation of a kit for predicting the therapeutic effect of an anti-PD-1 antibody and/or a CDK4/6 inhibitor on cancer. Such agents include, but are not limited to, those conventionally used in assays, including but not limited to, primers, probes, agents required for PCR, etc. The cancer is preferably melanoma. Preferably, the predicting comprises: the presence of a *BRAF* gene mutation indicating that the subject is suitable for treatment with an anti-PD-1 antibody, the presence of an *NRAS* gene mutation indicating that the subject is not suitable for treatment with an anti-PD-1 antibody, and the presence of a *CDK4* or *CCND1* gene amplification indicating that the subject is suitable for treatment with an anti-PD-1 antibody and a CDK4/6 inhibitor or for treatment with a CDK4/6 inhibitor alone.

### Abbreviation

The following abbreviations are used throughout the description and examples of the present invention:
- BID: One dose, twice a day
- CDR: Complementarity determining region
- DFS: Disease-free survival
- FR: Framework region
- IgG: Immunoglobulin G
- IHC: Immunohistochemistry
- OR: Overall response

- ORR: Objective response rate
- OS: Overall survival
- PD: Progressive disease
- PFS: Progression-free survival
- PR: Partial response
- CR: Complete response
- SD: Stable disease
- DLT: Dose-limiting toxicity
- MTD: Maximum tolerated dose
- AE: Adverse event
- Q2W: One dose every 2 weeks
- QD: One dose everyday
- CSD: Chronic sun-damaged
- non-CSD: Non-chronic sun-damaged
- IRC: Independent review committee
- TRAE: Treatment-related adverse event
- SAE: Serious adverse event
- MM: Melanoma

The present invention is further illustrated by the following examples, which should not be construed as limiting the present invention. The contents of all references cited throughout the present application are explicitly incorporated herein by reference.

### Examples

### Example 1. Clinical Study on Anti-PD-1 Antibody Alone for Treatment of Melanoma

Inclusion criteria: eligible subjects must (1) be at least 18 years old, (2) have locally advanced or metastatic melanoma, (3) be refractory to standard systemic therapy, (4) have an ECOG score of 0 or 1, (5) have no history of autoimmune disease or persistent infection, (6) have not previously received any anti-PD-1/or anti-PD-L1 immunotherapy.

Subjects must have an evaluable lesion according to RECIST v1.1 criteria, be not allowed to be treated with an anti-tumor drug at the same time, be not allowed to be treated with a systemic steroid drug or have not been treated with anti-CTLA4, anti-PD-1 or anti-PD-L1 antibodies.
Test drug: the anti-PD-1 antibody toripalimab (WO2014206107).
The dosage of the anti-PD-1 antibody used in this study: 3 mg/kg, i.v., once every two weeks (Q2W).

### Study design:

This is a single-arm, phase II, non-blind clinical trial. This study was conducted to evaluate the safety and anti-tumor activity of the anti-PD-1 antibody for treating patients with advanced melanoma.

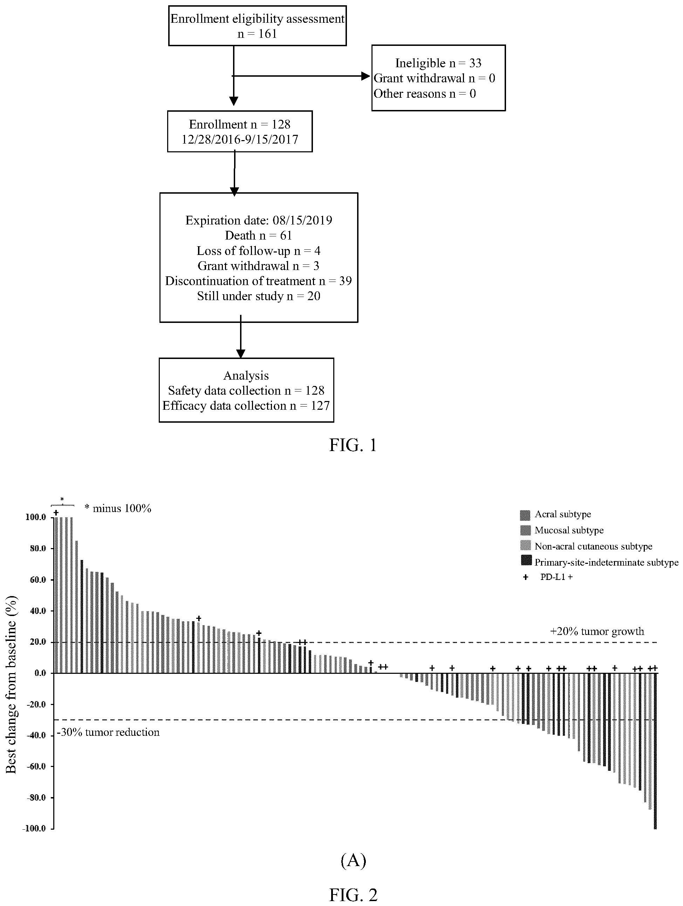

From Dec. 26, 2016 to Sept. 15, 2017, 161 patients with melanoma who had failed to respond to standard therapy were screened at total of six centers, and a total of 128 patients were enrolled in the study (as shown in FIG. 1). Demographics of the enrolled subjects are shown in Table 1. The average age was 52.5 years old, with 57 males (45.5%) and 71 females (55.5%). One patient was later diagnosed with cancer that had not recurred and was excluded from the efficacy analysis.

For melanoma subtypes, 50 cases (39.4%) were of the acral subtype, 22 cases (17.3%) were of the mucosal subtype, 29 cases (22.9%) were of the non-acral cutaneous subtype, and 26 cases (20.5%) were of the primary-site-indeterminate subtype. The *BRAF* mutation was recorded in 34 patients (26.6%) enrolled. Most of the patients have received previous multi-line therapies, with 88 (68.7%) patients experienced at least two previous systemic therapies. 99 (78.0%) patients have received previous systemic chemotherapy and 9 (7.0%) patients have received previous ipilimumab therapy.

**Table 1: Demographics of the enrolled subjects**

| Characteristics | Numerical value | N (100%) |
|---|---|---|
| Age | N | 127 (0) |
| | Average value | 52.49 ± 11.46 |
| | M(Q1-Q3) | 52.00 (45.00, 61.00) |
| | Min-max | 21.00, 76.00 |
| Gender | Male | 57 (44.88) |
| | Female | 70 (55.12) |
| ECOG | 0 | 73 (57.48) |
| | 1 | 54 (42.52) |
| TNM stage | III | 14 (11.02) |
| | IV M1a | 26(20.47) |
| | IV M1b | 51(40.16) |
| | IV M1c | 36(28.35) |
| Melanoma subtypes | Acral subtype | 50 (39.97) |
| | Mucosal subtype | 22 (17.32) |
| | Non-acral cutaneous subtype | 29 (22.83) |
| | Primary-unknown subtype | 26 (20.48) |
| PD-L1 status | Positive | 26 (20.47) |
| | Negative | 84 (66.14) |
| | Unknown | 17 (13.39) |
| LDH baseline | LDH<140 U/L | 5 (3.94) |
| | 140U/L≤LDH≤280U/L | 102 (80.31) |
| | LDH>280 U/L | 20 (15.75) |
| Previous therapy line | 1 | 40 (31.50) |
| | 2 | 31 (24.41) |
| | 3+ | 56 (44.096) |
| *BRAF* mutation | No | 86 (67.72) |
| | Yes | 34 (26.77) |
| | Unknown | 7 (5.51) |
| Previous ipilimumab therapy | No | 118 (92.91) |
| | Yes | 9 (7.09) |
| Previous chemotherapy | No | 28(22.0) |
| | Yes | 99(78.0) |

| | | |
|---|---|---|
| Note: PD-L1 positive status is defined as ≥ 1% expression of PD-L1 in tumor cells by SP142 IHC staining. | | |

### 1.1 Safety study

As of Aug. 15, 2019, i.e., 23 months after the last patient was enrolled, the average number of doses of toripalimab that patients received was 10 (ranging from 1 to 73). Of 128 patients, 116 (97.7%) experienced treatment-related side effects, most of which were grade 1 or 2. Common (> 5%) treatment-related side effects are shown in Table 2. Treatment-related SAEs occurred in 10 (7.8%) patients. Twenty-five (19.5%) patients experienced TRAEs of grade 3 or higher, including 13 (10.2%) grade 3 and 12 (9.4%) grade 4 side effects, and no patients experienced grade 5 side effects. 15 (11.7%) patients discontinued treatment due to TRAE; 8 (6.3%) patients experienced dose deferral due to TRAE. Less than 5% TRAEs of particular interest occurred, including 5 (3.9%) cases of leucoderma, 3 (2.3%) cases of liver injury (2 cases of grade 4), 2 (1.6%) cases of acute pancreatitis, 2 (1.6%) cases of interstitial lung disease (1 case of grade 3), 2 (1.6%) cases of adrenal insufficiency, 2 (1.6%) cases of hypopituitarism, and 1 (0.8%) case of uveitis (grade 3).

**Table 2. Common (≥ 20%) adverse events related to toripalimab therapy in all subjects (n = 36)**

| N (%) | All | Grade 1 | Grade 2 | Grade 3 | Grade 4 | Grade 5 |
|---|---|---|---|---|---|---|
| All adverse events | 125(97.7 | 42(32.8) | 42(32.8) | 29(22.7) | 12(9.4) | 0 |
| Elevated TSH | 42(32.8 | 34(26.6) | 8(6.3) | 0 | 0 | 0 |
| Elevated ALT | 41(32.0) | 34(26.6) | 5(3.9) | 2(1.6) | 0 | 0 |
| Hyperglycemia | 41(32.0) | 35(27.3) | 4(3.1) | 1(0.8) | 1(0.8) | 0 |
| Proteinuria | 35(27.3) | 33(25.8) | 1(0.8) | 1(0.8) | 0 | 0 |
| Hypothyroidism | 35(27.3) | 19(14.8) | 16(12.5) | 0 | 0 | 0 |
| Increased creatine kinase | 33(25.8) | 26(20.3) | 3(2.3) | 2(1.6) | 2(1.6) | 0 |
| Rash | 30(23.4) | 27(21.1) | 3(2.3) | 0 | 0 | 0 |
| Elevated AST | 30(23.4) | 28(21.9) | 1(0.8) | 1(0.8) | 0 | 0 |
| Skin depigmentation | 30(23.4) | 28(21.9) | 2(1.6) | 0 | 0 | 0 |
| Leukocyturia | 29(22.7) | 27(21.1) | 1(0.8) | 1(0.8) | 0 | 0 |
| Hematuresis | 29(22.7) | 29(22.7) | 0 | 0 | 0 | 0 |
| Leukopenia | 28(21.9) | 22(17.2) | 6(4.7) | 0 | 0 | 0 |
| Anemia | 27(21.1) | 9(7.0) | 15(11.7) | 3(2.3) | 0 | 0 |
| Amylase increase | 25(19.5) | 15(11.7) | 5(3.9) | 2(1.6) | 3(2.3) | 0 |
| Neutropenia | 24(18.8) | 18(14.1) | 6(4.7) | 0 | 0 | 0 |
| Pruritus | 24(18.8) | 20(15.6) | 3(2.3) | 1(0.8) | 0 | 0 |
| Hyperthyroidism | 23(18.0) | 18(14.1) | 5(3.9) | 0 | 0 | 0 |
| Elevated DBIL | 22(17.2) | 15(11.7) | 7(5.5) | 0 | 0 | 0 |
| Elevated TBIL | 22(17.2) | 22(17.2) | 0 | 0 | 0 | 0 |
| Reduced TSH | 19(14.8) | 16(12.5) | 3(2.3) | 0 | 0 | 0 |
| Fatigue | 19(14.8) | 19(14.8) | 0 | 0 | 0 | 0 |
| Loss of appetite | 19(14.8) | 17(13.3) | 2(1.6) | 0 | 0 | 0 |
| Fever | 15(11.7) | 15(11.7) | 0 | 0 | 0 | 0 |
| Cough | 15(11.7) | 12(9.4) | 3(2.3) | 0 | 0 | 0 |
| Elevated blood lipid | 14(10.9) | 2(1.6) | 5(3.9) | 6(4.7) | 1(0.8) | 0 |

### 1.2 Anti-tumor activity study

As of Aug. 15, 2019, in the intention to treat (ITT) population (n = 127), 61 (48.0%) patients died, 46 (36.2%) patients discontinued treatment, and 20 (15.7%) patients were still under study. The median duration of treatment was 4.6 months (ranging from 0.2 to 33.6 months). Of the 127 patients evaluated by IRC/RECIST v1.1, 22 patients achieved confirmed objective response (1 case of complete response and 21 cases of partial response). The ORR per RECIST v1.1 was 17.3% (95% CI 11.2-25.0), and the ORR per irRECIST was 18.1% (95% CI 11.8-25.9). The specific results are shown in Table 3.

**Table 3. Evaluation of clinical efficacy according to RECIST v1.1 or irRECIST criteria**

| | IRC | | Investigator | |
|---|---|---|---|---|
| | RECIST1.1 | irRECIST | RECIST1.1 | irRECIST |
| CR | 1(0.79) | 1(0.79) | 1(0.79) | 1(0.79) |
| PR | 21(16.54) | 22(17.32) | 23(18.11) | 24(18.90) |
| uPR | 3(2.36) | 2(1.57) | 2(1.57) | 2(1.57) |
| SD | 48(37.80) | 51(40.16) | 44(34.65) | 49(38.58) |
| PD | 48(37.80) | 43(33.86) | 52(40.94) | 44(34.65) |
| NE | 6(4.72) | 8(6.30) | 5(3.94) | 7(5.51) |
| Total | 127(100.00) | 127(100.00) | 127(100.00) | 127(100.00) |
| ORR(%) 95% CI | 17.32(11.19, 25.04) | 18.11(11.84, 25.92) | 18.90(12.50, 26.80) | 19.69(13.16, 27.67) |
| DCR(%) 95% CI | 57.48(48.40, 66.20) | 59.84(50.78, 68.44) | 55.12(46.041, 63.95) | 59.84(50.78, 68.44) |

| | | | | |
|---|---|---|---|---|
| Note: ORR = (CR + PR)/total × 100%; DCR = (CR + PR + uPR + SD)/total × 100%. CR: complete response; PR: partial response; uPR: unconfirmed PR; SD: stable disease; PD: progressive disease; NE: not evaluated; ORR: objective response rate; DCR: disease control rate; and CI: confidence interval. | | | | |

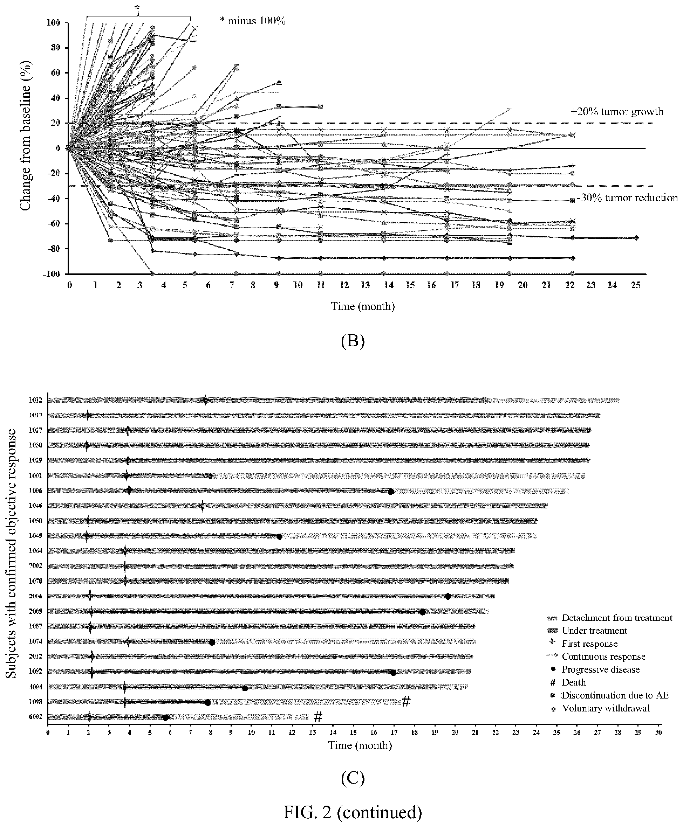

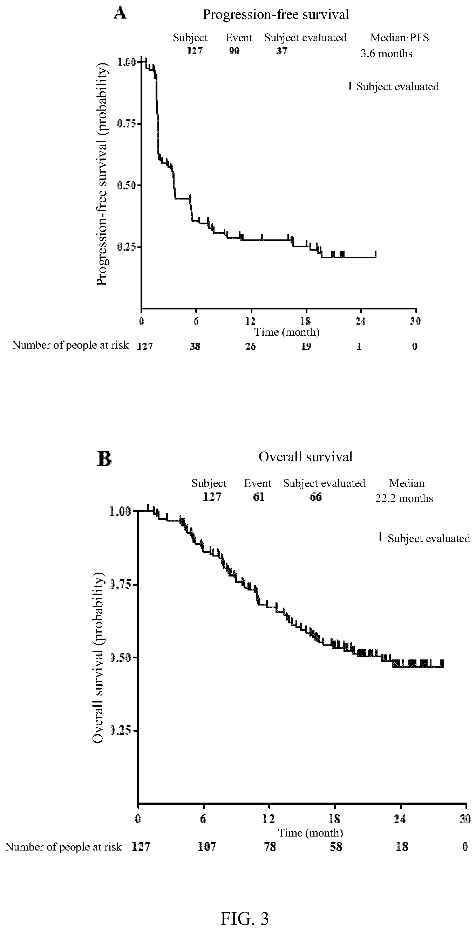

The size of the target lesion was reduced relative to baseline in 49 (38.6%) subjects. The specific results are shown in FIG. 2(A). Three SD subjects initially showed partial responses, but these responses could not be confirmed. The median duration of response was 3.5 months (95% CI 1.7-3.6), the median duration of response (DOR) was not reached (NR), and progressive disease occurred after the initial response in only 9 of 22 patients. The specific results are shown in FIG. 2(B) and FIG. 2(C). The DCR per RECIST v1.1 was 57.5% (95% CI 48.4-66.2) and the DCR per irRECIST was 59.8% (95% CI 50.8-68.4). The median PFS per RECIST v1.1 was 3.6 months (95% CI 3.7-5.5), and the median PFS per irRECIST was 3.7 months (95% CI 3.3-9.1). The specific results are shown in FIG. 3(A).

The median overall OS for the ITT population (n = 127) was estimated to be 22.2 months (95% CI 15.3 to NR). The specific results are shown in FIG. 3(B).

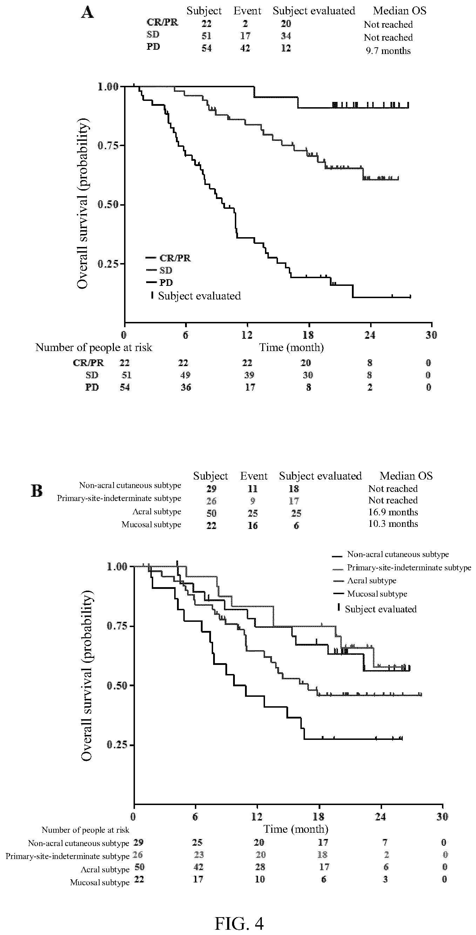

The median OS for subjects who experienced objective response (n = 22) and stable disease (n = 51) was NR, with only 2 deaths among 22 PR/CR patients, 17 deaths among 51 SD patients, and the median overall survival mOS for 54 patients with progressive disease was 9.7 months. The specific results are shown in FIG. 4(A).

For the four melanoma subtypes, i.e., acral, mucosal, non-acral cutaneous and primary-site-indeterminate subtypes, the ORRs per RECIST v1.1 were 14.0%, 0%, 31.0% and 23.1%, respectively, and the median PFSs per RECIST v1.1 were 3.2, 1.9, 5.5 and 7.3 months, respectively, as evaluated by the independent review committee (IRC). The specific results are shown in Table 4 and FIG. 5. The median OSs for the acral and mucosal subtypes were 16.9 months and 10.3 months, respectively, while the median OSs for the primary-site-indeterminate and non-acral cutaneous subtypes were NR by the expiration date. The specific results are shown in FIG. 4(B).

**Table 4. Evaluation of clinical efficacy and survival in melanoma subtypes by RECIST v1.1**

| | **Total** | **Acral subtype** | **Mucosal subtype** | **Non-acral cutaneous subtype** | **Primary-unknown subtype** |
|---|---|---|---|---|---|
| Total n | 127 | 50 | 22 | 29 | 26 |

| ORR (%) | | | | | |
|---|---|---|---|---|---|
| IRC/RECIST v1.1 | 17.3 | 14.0 | 0 | 31.0 | 23.1 |
| PI/ RECIST v1.1 | 18.9 | 14.0 | 9.1 | 31.0 | 19.2 |

| DCR (%) | | | | | |
|---|---|---|---|---|---|
| IRC/RECIST v1.1 | 57.5 | 52.0 | 40.9 | 65.5 | 73.1 |
| PI/RECIST v1.1 | 55.9 | 52.0 | 36.4 | 69.0 | 65.4 |
| mPFS (month) evaluated by IRC (95% CI) | 3.6(2.2, 5.3) | 3.2 (1.8, 3.6) | 1.9 (1.8, 5.3) | 5.5 (1.9, 19.2) | 7.3 (3.5, 18.4) |
| Limited, n (%) | 39 (30.7) | 12 (24.0) | 7 (31.8) | 10 (34.5) | 10 (38.5) |
| Event, n (%) | 88 (69.3) | 38 (76.0) | 15 (68.2) | 19 (65.5) | 16 (61.5) |
| Median OS (month) (95% CI) | 22.2 (15.3, NE) | 16.9(10.9, NE) | 10.3(6.6, 16.5) | NE(15.3, NE) | NE(19.5, NE) |
| Limited, n (%) | 66 (52.0) | 25 (50.0) | 6 (27.3) | 18 (62.1) | 17 (65.4) |
| Death, n (%) | 61 (48.0) | 25 (50.0) | 16 (72.7) | 11 (37.9) | 9 (34.6) |

| | | | | | |
|---|---|---|---|---|---|
| NE: not evaluable. | | | | | |

### 1.3 Pharmacokinetics and immunogenicity

The steady-state mean trough plasma concentration for toripalimab was 39.8 µg/mL (range of 4.9-92.4 µg/mL), which was much higher than the full PD-1 blocking concentration of 1.5 µg/mL (10 nmol/L). 128 patients were tested for anti-drug antibodies (ADAs). Sixteen patients (12.5%) were ADA positive, of which 3 were positive before treatment and 13 (10.2%) were positive after treatment. However, there were no continuous positive samples from ADA positive individuals. Only 1 of 16 patients had a reduction in trough concentration of toripalimab with the detection of ADA, indicating the presence of neutralizing activity. There was no significant difference between ADA-positive and ADA-negative patients in AE, SAE, incidence of grade 3 and above AE, discontinuation or dose deferral, and clinical efficacy.

### Example 2. Study on Correlation Between Biomarkers and Clinical Efficacy

### 2.1 Expression of PD-L1 in tumors

The correlation between tumor histology and the clinical efficacy of the anti-PD-1 antibody was analyzed by detecting biopsy specimens from 127 patients. The detection was performed using rabbit anti-human PD-L1 antibody SP142 from Roche. PD-L1 positive status is defined as the presence of tumor cells with membrane staining intensity ≥ 1%.

As shown in FIG. 6(A), 26 (20.5%) patients were PD-L1 positive, 84 (66.1%) patients were PD-L1 negative, and 17 (13.4%) patients showed unknown PD-L1 expression. As shown in FIG. 6(B), for the four melanoma subtypes, the proportion of PD-L1⁺ status occurred in the acral subtype (6.8%) and the mucosal subtype (10.5%) was significantly lower than that occurred in the non-acral subtype (37.5%) and primary-site-indeterminate subtype (52.2%).

As shown in FIG. 6(C) and FIG. 6(D), PD-L1⁺ patients had a better response to toripalimab therapy in ORR (38.5% vs. 11.9%, *p* = 0.0065) and DCR (80.8% vs. 48.8%, *p* = 0.006) than PD-L1⁻ patients. PD-L1⁺ patients also had a significant survival advantage over PD-L1⁻ patients in progression-free survival PFS and overall survival OS, with median PFS of 7.7 months vs. 2.7 months, HR = 0.53 (95% CI 0.32-0.88), *p* = 0.013; and with median OS of NR vs. 14.4 months, HR = 0.35 (95% CI 0.19-0.63), *p* = 0.0005.

### 2.2 Tumor mutation burden (TMB) assay

In the experiment of Example 1, whole exome sequencing was performed on tumor biopsy specimens and matched peripheral blood samples from patients using second generation sequencing techniques. Tumor mutation burden (TMB) was determined by analyzing somatic mutations within coding regions of the human genome. As shown in FIG. 6(A), 98 patients achieved valid results, and TMB was generally low in this study, with a median TMB of 1.5 mutations per million base pairs (Muts/Mb). No patients with high MSI were found. TMB exceeded 10 Muts/Mb in only 6 patients, and exceeded 20 Muts/Mb in 3 of them.

As shown in FIG. 6(B), of the four melanoma subtypes, the mucosal melanoma had the lowest TMB, with a median TMB of 1.6 Muts/Mb, and only one patient (6.7%) had a TMB greater than 3.6 Muts/Mb. As suggested by Robert M. Samstein et al., a cut-off value (3.6 muts/Mb) of the top 20% of TMB values was selected for this study to define the high TMB population. With 3.6 Muts/Mb as a cut-off value, patients with TMB ≥ 3.6 Muts/Mb (n = 20) had a better response than patients with TMB < 3.6 Muts/Mb (n = 78) (ORR 30.0% vs. 12.8%), but the difference was not statistically significant (p =0.088). The specific results are shown in FIG. 6(A) and Table 5. The group with TMB ≥ 3.6 Muts/Mb also had higher PFS and OS values, but the difference was not statistically significant either (FIG. 6, E and F).

The group with TMB ≥ 3.6 Muts/Mb (n = 20) and the PD-L1⁺ group (n = 26) were mainly two independent groups in this study, and only 7 of the 26 PD-L1 positive patients had TMB ≥ 3.6 Muts/Mb (FIG. 6, A).

**Table 5. Cut-off value of top 20% of TMB values of each subtype used for efficacy analysis**

| | Non-acral subtype | Acral subtype | Mucosal subtype | Primary-site-indeterminate subtype | All |
|---|---|---|---|---|---|
| Number of subjects | 23 | 39 | 15 | 21 | 98 |
| Total ORR | 30.4% | 10.3% | 0% | 23.8% | 16.3 % |
| Top 20% TMB (Muts/Mb) | 3.6 | 4.2 | 3.2 | 4.2 | 3.6 |
| ORR of the top 20% of TMB | 40.0% | 25.00% | 0% | 50.0% | 30.0 % |
| ORR of last 80% of TMB | 27.8% | 6.5% | 0% | 17.6% | 12.8 % |
| *p* value of top 20% vs. last 80% | 0.62 | 0.18 | 1.00 | 0.23 | 0.08 8 |

### Example 3. Gene Sequencing Analysis

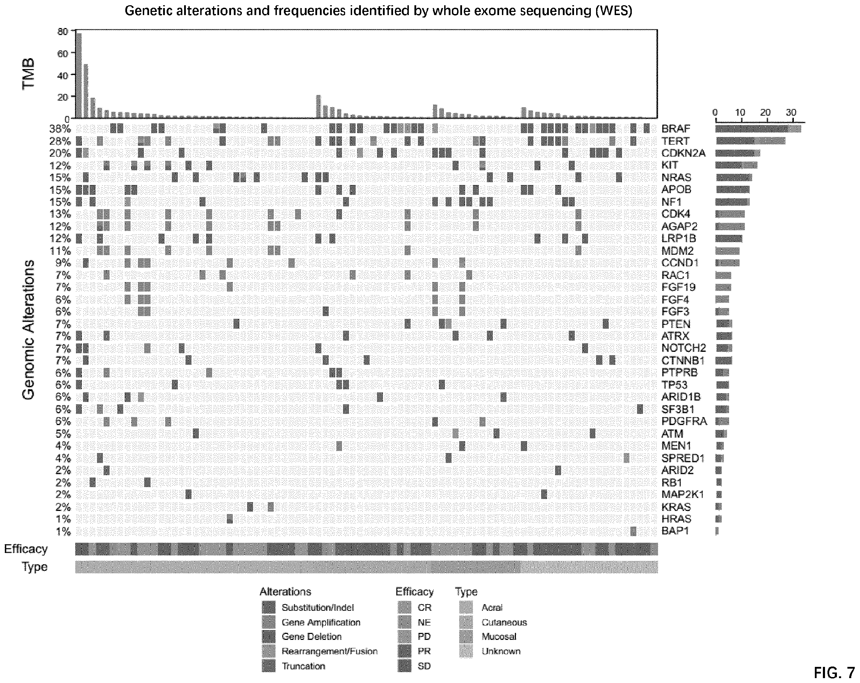

In the experiment of Example 1, whole exome sequencing was performed on tumor biopsy specimens and matched peripheral blood samples from patients using second generation sequencing techniques, and 19278 gene mutations were identified from 98 patients, including 7964 missense mutations, 509 gene deletions, 482 gene rearrangements, 288 alternative splice sites, 129 frameshift truncations, and 8157 gene amplifications. After excluding genes that were frequently mutated in the public exome, the most frequently altered genes (≥ 10%) were *BRAF* (33%), *TERT(32%), CDKN2A* (12%), *NRAS* (16%), *CDK4* (12%), *APOB* (11%), *CCND1* (11%), *AGAP2* (11%), *NF1* (10%), *LRP1B* (10%), *MDM2* (10%) and *KIT* (10%). The specific results are shown in FIG. 7.

As shown in Table 6, sequencing results from 98 patients showed that genomic alterations have a unique pattern in the melanoma subtypes. The *BRAF* mutation occurred more frequently in non-acral cutaneous subtype (11/23, 48%) and primary-unknown subtype (14/21, 67%), and less frequently in acral subtype (7/39, 18%) and mucosal subtype (0/15, 0%). In contrast, the *NF1* mutation was enriched in mucosal subtype (4/15, 27%) to a greater extent than the other three subtypes (8%, 4% and 10%). Furthermore, the *CDK4* or *CCND1* (Cyclin D1) amplification was observed in 33% (13/39) of the acral subtype and 20% (3/15) of the mucosal subtype, but only in 9% (2/23) of the non-acral cutaneous subtype and 10% (2/21) of the primary-unknown subtype. It was found in this study that 70% (7/10) of the *CCND1* copy number variations were observed in the acral subtype as part of the 11q13 gene amplification. Subjects with *CCND1* amplification (n = 10) had a low response (ORR 0%) to toripalimab therapy. The study suggested that for patients with *CCND1* amplification, CDK4/6 targeted therapy or CDK4/6 inhibitors in combination with anti-PD-1 antibodies would have better potential.

It was also found in this study that subjects with *NRAS* mutation (n = 16), including 7 patients with acral melanoma, 3 patients with mucosal melanoma, 3 patients with non-acral (cutaneous) melanoma, and 3 patients with melanoma with an unknown primary, had an ORR of 6.3% (1/16) for toripalimab therapy. The study suggested that the *NRAS* mutation can be used as a predictor of poor prognosis for immunotherapy.

**Table 6. Whole exome sequencing of gene mutations from 98 patients**

| N (%) | *BRAF* | *NF1* | *KIT* | *NRAS* | *CCND1* |
|---|---|---|---|---|---|
| Non-acral cutaneous subtype n = 23 | 11 (48) | 1 (4) | 0 | 3 (13) | 1(4) |
| Primary-site-indeterminate subtype n =21 | 14 (67) | 2 (10.0) | 2 (10.0) | 3 (14) | 1(5) |
| Acral subtype n = 39 | 7 (18) | 3 (8) | 6 (15) | 7 (18) | 7 (18) |
| Mucosal subtype n = 15 | 0 (0) | 4 (27) | 2 (13) | 3 (20) | 1 (7) |
| Total n = 98 | **32(33)** | **10 (10)** | **10 (10)** | **16 (16)** | **10 (10)** |

### Example 4. Messenger RNA Expression Profiling in Tumor Biopsy

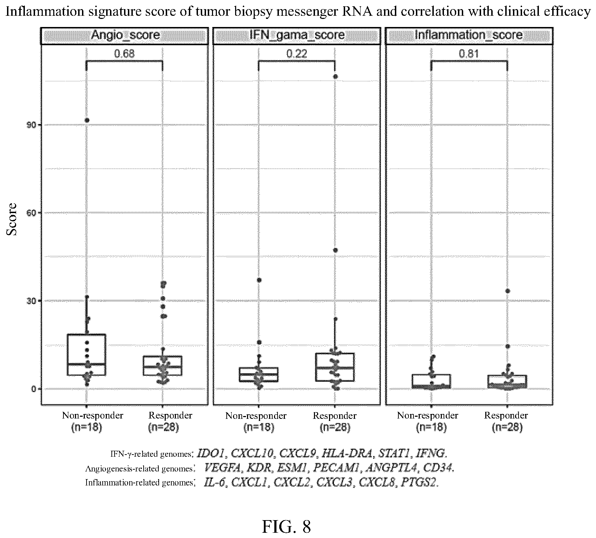

In the experiment of Example 1, mRNA extracted from tumor biopsies was subjected to RNA sequencing and expression profiling. Valid results were obtained from 46 patients. The gene expression profiles of IFN-γ-related genomes *(IDOl, CXCL10, CXCL9, HLA-DRA, STAT1, IFNG*), inflammation-related genomes (*IL-6, CXCL1, CXCL2, CXCL3, CXCL8, PTGS2),* and angiogenesis-related genomes *(VEGFA, KDR, ESM1, PECAM1, ANGPTL4, CD34)* were evaluated. No significant differences were found between patients with clinical benefits (CR + PR + SD) and patients with progressive disease in signature scores for the angiogenesis-related genomes, IFN-related genomes and inflammation-related genomes (FIG. 8).

## Claims

1. Use of an anti-PD-1 antibody or an antigen-binding fragment thereof in the preparation of a medicament for treating a patient with cancer, wherein the patient with cancer is a patient with cancer who has positive PD-L1 expression, or a patient with cancer who has tumor mutation burden (TMB) of ≥ 3.6 Muts/Mb in peripheral blood or tumor tissue; or a patient with cancer who has both positive PD-L1 expression and tumor mutation burden of ≥ 3.6 Muts/Mb in peripheral blood or tumor tissue; or a patient with cancer who has a *BRAF* gene mutation detected in tumor tissue.

2. The use according to claim 1, wherein the patient with cancer is a patient with melanoma.

3. The use according to claim 2, wherein the melanoma is advanced or metastatic melanoma.

4. The use according to claim 2, wherein the patient with melanoma is selected from a patient with acral melanoma, a patient with mucosal melanoma, a patient with non-acral cutaneous melanoma, and a patient with melanoma with indeterminate primary site, preferably a patient with non-acral melanoma or a patient with melanoma with indeterminate primary site.

5. Use of a CDK4/6 inhibitor alone or in combination with an anti-PD-1 antibody or an antigen-binding fragment thereof in the preparation of a medicament for treating a patient with cancer, wherein the patient with cancer is a patient with cancer who has a *CDK4* or *CCND1* gene amplification detected in tumor tissue.

6. The use according to any one of claims 1 to 5, wherein the anti-PD-1 antibody comprises light chain complementarity determining regions (LCDRs) and heavy chain complementarity determining regions (HCDRs), wherein the LCDRs comprise amino acid sequences set forth in SEQ ID NOs: 1, 2 and 3, and the HCDRs comprise amino acid sequences set forth in SEQ ID NOs: 4, 5 and 6.

7. The use according to claim 6, wherein the anti-PD-1 antibody comprises a light chain variable region (VL) and a heavy chain variable region (VH), wherein the VL comprises an amino acid sequence set forth in SEQ ID NO: 7, and the VH comprises an amino acid sequence set forth in SEQ ID NO: 8.

8. The use according to claim 6, wherein the anti-PD-1 antibody is an anti-PD-1 antibody comprising a light chain and a heavy chain, wherein the light chain comprises an amino acid sequence set forth in SEQ ID NO: 9, and the heavy chain comprises an amino acid sequence set forth in SEQ ID NO: 10.

9. The use according to any one of claims 1 to 5, wherein the anti-PD-1 antibody is selected from one or more of nivolumab, pembrolizumab, toripalimab, sintilimab, camrelizumab, tislelizumab and cemiplimab, preferably toripalimab.

10. The use according to any one of claims 1 to 5, wherein the anti-PD-1 antibody is a monoclonal antibody or an antigen-binding fragment thereof.

11. The use according to claim 5, wherein the CDK4/6 inhibitor includes palbociclib, ribociclib or abemaciclib.

12. The use according to any one of claims 1 to 11, wherein the anti-PD-1 antibody or the antigen-binding fragment thereof is administered at a single dose of about 0.1 mg/kg to about 10.0 mg/kg body weight, e.g., about 0.1 mg/kg body weight, about 0.3 mg/kg body weight, about 1 mg/kg body weight, about 2 mg/kg body weight, about 3 mg/kg body weight, about 5 mg/kg body weight or 10 mg/kg body weight, or selected from a fixed dose of about 120 mg to about 480 mg, e.g., a fixed dose of about 120 mg, 240 mg, 360 mg or 480 mg.

13. The use according to claim 12, wherein the anti-PD-1 antibody or the antigen-binding fragment thereof is administered at a frequency of about once every week, once every two weeks, once every three weeks, once every four weeks or once a month, preferably once every two weeks.

14. The use according to claim 12, wherein the anti-PD-1 antibody or the antigen-binding fragment thereof is administered at a single dose of 1 mg/kg body weight, 3 mg/kg body weight or 10 mg/kg body weight, or of a fixed dose of 240 mg or 480 mg once every two weeks.

15. The use according to claim 12, wherein the anti-PD-1 antibody or the antigen-binding fragment thereof is administered parenterally, e.g., by intravenous infusion, in a liquid dosage form, e.g., an injection.

16. The use according to claim 12, wherein the anti-PD-1 antibody or the antigen-binding fragment thereof can be administered in cycles of one week, two weeks, three weeks, one month, two months, three months, four months, five months, half a year or longer; optionally, the cycles each can be identical or different, and at identical or different intervals.

17. The use according to any one of claims 1 to 8, wherein the patient with cancer is a patient with melanoma who has a *BRAF* gene mutation detected in tumor tissue, and the anti-PD-1 antibody is toripalimab.

18. The use according to claim 5, wherein the use is use of an anti-PD-1 antibody or an antigen-binding fragment thereof in combination with a CDK4/6 inhibitor, wherein the anti-PD-1 antibody is toripalimab, and the CDK4/6 inhibitor includes palbociclib, ribociclib or abemaciclib.

19. A kit for predicting the therapeutic effect of an anti-PD-1 antibody administered alone to a patient with cancer, wherein the kit comprises: an agent for detecting a *BRAF* gene mutation in cancer tissue or peripheral blood of the patient, or an agent for detecting an *NRAS* gene mutation in cancer tissue or peripheral blood of the patient, or an agent for detecting a *CDK4* or *CCND1* gene amplification in cancer tissue or peripheral blood of the patient.

20. Use of an agent for detecting a *BRAF* mutation in cancer tissue or peripheral blood of a patient, or an agent for detecting an *NRAS* mutation in cancer tissue or peripheral blood of a patient, or an agent for detecting a *CDK4* or *CCND1* gene amplification in cancer tissue or peripheral blood of a patient in the preparation of a kit for predicting the therapeutic effect of an anti-PD-1 antibody or an antigen-binding fragment thereof on melanoma.
